# EUROPEAN PATENT APPLICATION

(11) **EP 2 441 832 A1**
(43) Date of publication of application: **18.04.2012**
(21) Application number: 10786244.3
(22) Date of filing: 11.06.2010
(51) Int. Cl.: C12N 15/09, C07K 16/40, C12N 1/15, C12N 1/19, C12N 1/21, C12N 5/10, C12N 9/10, C12N 9/24

(54) **NOVEL PROTEIN AND GENE THAT CODES THEREFOR**

(30) Priority: 12.06.2009 JP 2009141312
(71) Applicant: Japan Tobacco, Inc., Tokyo 105-8422 (JP)
(72) Inventor: MINE, Toshiki, Iwata-shi Shizuoka 438-0802 (JP); YAMAMOTO, Takeshi, Iwata-shi Shizuoka 438-0802 (JP); KAJIWARA, Hitomi, Iwata-shi Shizuoka 438-0802 (JP); TSUKAMOTO, Hiroshi, Iwata-shi Shizuoka 438-0802 (JP)
(74) Representative: Behnisch, Werner
(86) International application number: PCT/JP2010/059952
(87) International publication number: WO 2010/143713

(57) **Abstract**

The present invention provides a novel protein having neuraminidase activity and/or β-galactoside-α2,6-sialyltransferase activity and a nucleic acid encoding the protein. The present invention further provides a vector containing a nucleic acid encoding the protein, a host cell transformed with the vector, together with a method for producing a recombinant β-galactoside-α,2,6-sialyltransferase. The present invention also provides an antibody specifically recognizing the protein.

## Description

### TECHNICAL FIELD

The present invention relates to a protein having neuraminidase activity and/or β-galactoside-α2,6-sialyltransferase activity, a nucleic acid encoding the protein, a method for producing the enzyme using a microorganism that has been transformed with the gene encoding the protein, and an antibody specifically recognizing the protein.

### BACKGROUND ART

Sugar chains are compounds composed of various sugars, for example, monosaccharides such as galactose and *N*-acetylglucosamine. The sugar chains of glycoproteins or glycolipids (hereinafter referred to as sugar chains of complex carbohydrates) have very important functions *in vivo.* Past studies suggest that sugar chains containing sialic acid, which is a monosaccharide, among sugar chains, particularly expresses an important function. For example, mainly in mammalian cells, it is shown that sugar chains containing sialic acid are important molecules functioning in intracellular and cell-extracellular matrix signaling in differentiation and development, and functioning as tags of complex carbohydrates, and that complex carbohydrates containing sialic acid are prominently involved in formation of synapses in brain and nerve cells, neurological development, and thus improvements in learning ability. Sialic acid is a general term for acyl derivatives of neuraminic acid, and one of the acidic monosaccharides having a carboxyl group in its structure. Until now, 50 or more molecular species have been identified, mainly in mammals, echinoderms, and bacteria. Typical examples of the sialic acids that are known include *N*-acetylneuraminic acid (Neu5Ac), *N*-glycolylneuraminic acid (Neu5Gc), and deaminoneuraminic acid (KDN). Among these sialic acids, only *N*-acetylneuraminic acid is generally found in human body. It is known in a specific example that *N-*glycolylneuraminic acid is present in some cancer cells.

Neuraminidase (sialidase) is an enzyme catalyzing a reaction of releasing a sialic acid residue located at a nonreducing terminus of a sugar chain of complex carbohydrates. Until now, many neuraminidase proteins have been found in, for example, animals, microorganisms, and viruses, and genes encoding neuraminidase proteins have been cloned. Known linkage modes of sialic acid are the following three types, i.e., α2,3-linkage, α2,6-linkage, and α2,8-linkage. Most neuraminidases that have been reported cleave sialic acid from sugar chains of complex carbohydrates containing sialic acid regardless of the linkage mode of the sialic acid. There are a small number of exceptional examples that have reported on neuraminidases that semiselectively cleave sialic acid of which linkage mode is α2,3-linkage; however, there is no report on neuraminidase that selectively or preferentially cleaves sialic acid of which linkage mode is α2,6-linkage.

Glycosyltransferases are enzymes involved in biosynthesis of sugar chains of complex carbohydrates *in vivo.* The sialic acids found in sugar chains such as glycoproteins and glycolipids are transferred to sugar chains serving as sugar acceptor substrates by a group of glycosyltransferases called sialyltransferases.

The sialyltransferases that have been reported until now are classified into several groups, and many β-galactoside-α2,6-sialyltransferases and their genes derived from animals, in particular, mammals, have been reported (Hamamoto, T., et al., Bioorg. Med. Chem., 1, 141-145 (1993); Weinstein, J., et al., J. Biol. Chem., 262, 17735-17743 (1987)). These animal-derived enzymes show significantly high specificity to sugar acceptor substrates, and thus types of sugar chain structures in which sialic acid can be transferred *in vivo* are limited. That is, significantly limited glycoproteins and glycolipids can be generated by the reactions of sialyltransferases. On the other hand, as β-galactoside-α2,6-sialyltransferases and their genes derived from marine bacteria, those isolated from a microorganism belonging to *Photobacterium damselae* have been reported (International Publication No. WO98/38315; United States Patent No. 6255094; and Yamamoto, T., et al., J. Biochem., 120, 104-110 (1996)). It is known that these sialyltransferases derived from marine microorganisms have a significantly broad sugar acceptor substrate specificity compared to the above-mentioned animal-derived enzymes, so that sialic acid can be transferred to a sugar chain to which sialic acid cannot be transferred by the animal-derived sialyltransferase. That is, it can be expected that when a microorganism-derived sialyltransferase is expressed in animal cells, the structures of glycoproteins and glycolipids generated in the cells extend to an extremely broad range.

However, the optimum reaction temperature of many marine microorganism-derived sialyltransferases that have been known is 30°C or less, and it is shown that the enzyme activity is rapidly lost in a temperature range higher than that. In general, the optimum temperature for cultivating mammalian cells is about 37°C. This raises the following issue: When animal cells are transformed with an expression vector containing a marine bacterium-derived sialyltransferase and are allowed to grow in an environment suitable for the animal cells, the enzyme activity cannot be expressed even if a marine microorganism-derived sialyltransferase protein is expressed in the cells. Accordingly, there are demands for marine microorganism-derived sialyltransferases of which optimum reaction temperature is near the growth temperature of animal cells and for its genes. Furthermore, to reveal the function of a novel sugar chain containing sialic acid, synthesis of the sugar chain is important. However, synthesis of sugar chains still has many challenges. A possible countermeasure for solving the problems is to utilize a combination of various animal-derived glycosyltransferases of which a large number of genes were acquired and were expressed as recombinant enzymes until now with microorganism-derived sialyltransferase that has a broad sugar acceptor substrate specificity and an optimum temperature of about 37°C. However, no marine microorganism-derived sialic acid transferase having an optimum temperature of about 37°C has been reported until now.

### CITATION LIST

### PATENT DOCUMENT

Patent Document 1: International Publication No. WO98/38315
Patent Document 2: U.S. Patent No. 6255094

### NON-PATENT DOCUMENT

Non-Patent Document 1: Hamamoto, T., et al., Bioorg. Med. Chem., 1, 141-145 (1993)
Non-Patent Document 2: Weinstein, J., et al., J. Biol. Chem., 262, 17735-17743 (1987)
Non-Patent Document 3: Yamamoto, T., et al., J. Biochem., 120, 104-110 (1996)

### SUMMARY OF INVENTION

### TECHNICAL PROBLEM

It is an object of the present invention to provide a novel protein having neuraminidase activity and/or β-galactoside-α2,6-sialyltransferase activity, derived from a microorganism belonging to the genus Photobacterium of the family Vibrionaceae and to provide a nucleic acid encoding the protein. More specifically, it is an object of the present invention to provide a novel protein having neuraminidase activity that specifically cleaves sialic acid of α2,6-linkage and/or β-galactoside-α2,6-sialyltransferase activity having an optimum reaction temperature of 30°C to 40°C and to provide a nucleic acid encoding the protein.

### SOLUTION TO PROBLEM

The present inventors have characterized diligently 4000 or more microbial strains separated from everywhere in Japan and, as a result, have found a strain producing β-galactoside-α2,6-sialyltransferase activity in strains of microorganisms belonging to the genus Photobacterium. Then, the inventors have cloned a novel α2,6-sialyltransferase gene from this strain using a probe produced by reference to DNA sequence information of, for example, known β-galactoside-α2,6-sialyltransferase gene derived from a *Photobacterium damselae* JT0160 strain and β-galactoside-α2,6-sialyltransferase gene derived from *Photobacterium leiognathi* JT-SHIZ-145 strain. As a result of expressing this novel gene in *E. coli* cells, it has been found that this gene encodes a protein having β-galactoside-α2,6-sialyltransferase activity and that the encoded enzyme protein has an optimum reaction temperature of 30°C to 40°C. The inventors have purified this novel recombinant enzyme and analyzed it in detail and have found that this recombinant enzyme efficiently transfers sialic acid to a monosaccharide or a galactose or *N*-acetylgalactosamine residue in a sugar chain through α2,6-linkage and also has neuraminidase activity that specifically cleaves sialic acid of α2,6-linkage, and thereby have accomplished the present invention. The present invention provides a neuraminidase that is a novel β-galactoside-α2,6-sialyltransferase having an optimum reaction temperature of 35°C to 40°C and/or that specifically cleaves sialic acid of α2,6-linkage, a nucleic acid encoding the enzyme, and a method of producing a polypeptide having the enzyme activity.

Accordingly, the present invention is characterized as follows:

Aspect 1: An isolated protein comprising an amino acid sequence selected from the group consisting of SEQ ID NO: 2, SEQ ID NO: 4, and amino acids 16 to 511 of SEQ ID NO: 2.

Aspect 2: An isolated protein having neuraminidase activity and/or β-galactoside-α2,6-sialyltransferase activity, wherein the protein comprises:
(a) an amino acid sequence having deletion, substitution, insertion, and/or addition of one or more amino acids in an amino acid sequence selected from the group consisting of SEQ ID NO: 2, SEQ ID NO: 4, and amino acids 16 to 511 of SEQ ID NO: 2; or
(b) an amino acid sequence having an amino acid identity of 97% or more with an amino acid sequence selected from the group consisting of SEQ ID NO: 2, SEQ ID NO: 4, and amino acids 16 to 511 of SEQ ID NO: 2.

Aspect 3: An isolated protein encoded by a nucleic acid comprising a nucleotide sequence selected from the group consisting of SEQ ID NO: 1, SEQ ID NO: 3, and nucleotides 46-1536 of SEQ ID NO: 1.

Aspect 4: An isolated protein having neuraminidase activity and/or β-galactoside-α2,6-sialyltransferase activity, wherein the protein being encoded by a nucleic acid comprises:
(a) a nucleotide sequence having deletion, substitution, insertion, and/or addition of one or more nucleotides in a nucleotide sequence selected from the group consisting of SEQ ID NO: 1, SEQ ID NO: 3, and nucleotides 46-1536 of SEQ ID NO: 1;
(b) a nucleotide sequence having an identity of 97% or more with a nucleotide sequence selected from the group consisting of SEQ ID NO: 1, SEQ ID NO: 3, and nucleotides 46-1536 of SEQ ID NO: 1; or,
(c) a nucleotide sequence hybridizable under stringent conditions with the complementary strand of a nucleotide sequence selected from the group consisting of SEQ ID NO: 1, SEQ ID NO: 3, and nucleotides 46-1536 of SEQ ID NO: 1.

Aspect 5: The isolated protein according to any one of aspects 1 to 4, wherein the protein has neuraminidase activity, and wherein said neuraminidase activity is an activity selectively cleaves a sialic acid residue located at the nonreducing terminus of a sugar chain with α2,6-linkage.

Aspect 6: The isolated protein according to any one of aspects 1 to 4, wherein the protein has an optimum reaction pH of 5.0 to 7.0 for the neuraminidase activity.

Aspect 7: The isolated protein according to any one of aspects 1 to 4, wherein the protein has an optimum reaction temperature of 25°C to 40°C for the neuraminidase activity.

Aspect 8: The isolated protein according to any one of aspects 1 to 4, wherein the protein has an optimum reaction pH of 4.0 to 9.0 for the β-galactoside-α2,6-sialyltransferase activity.

Aspect 9: The isolated protein according to any one of aspects 1 to 4, wherein the protein has an optimum reaction temperature of 30°C to 40°C for the β-galactoside-α2,6-sialyltransferase activity.

Aspect 10: The isolated protein according to any one of aspects 1 to 4, wherein the protein is derived from a microorganism belonging to the genus Photobacterium.

Aspect 11: An isolated nucleic acid encoding a protein comprising an amino acid sequence selected from the group consisting of SEQ ID NO: 2, SEQ ID NO: 4, and amino acids 16 to 511 of SEQ ID NO: 2.

Aspect 12: An isolated nucleic acid encoding a protein having neuraminidase activity and/or β-galactoside-α2,6-sialyltransferase activity, which encodes a protein comprising:
(a) an amino acid sequence having deletion, substitution, insertion, and/or addition of one or more amino acids in an amino acid sequence selected from the group consisting of SEQ ID NO: 2, SEQ ID NO: 4, and amino acids 16 to 511 of SEQ ID NO: 2; or
(b) an amino acid sequence having an amino acid identity of 97% or more with an amino acid sequence selected from the group consisting of SEQ ID NO: 2, SEQ ID NO: 4, and amino acids 16 to 511 of SEQ ID NO: 2.

Aspect 13: The isolated nucleic acid comprising a nucleotide sequence selected from the group consisting of SEQ ID NO: 1, SEQ ID NO: 3, and nucleotides 46-1536 of SEQ ID NO: 1.

Aspect 14: An isolated nucleic acid encoding a protein having neuraminidase activity and/or β-galactoside-α2,6-sialyltransferase activity, wherein the nucleic acid comprises:
(a) a nucleotide sequence having deletion, substitution, insertion, and/or addition of one or more nucleotides in a nucleotide sequence selected from the group consisting of SEQ ID NO: 1, SEQ ID NO: 3, and nucleotides 46-1536 of SEQ ID NO: 1;
(b) a nucleotide sequence having an identity of 97% or more with a nucleotide sequence selected from the group consisting of SEQ ID NO: 1, SEQ ID NO: 3, and nucleotides 46-1536 of SEQ ID NO: 1; or,
(c) a nucleotide sequence hybridizable under stringent conditions with the complementary strand of a nucleotide sequence selected from the group consisting of SEQ ID NO: 1, SEQ ID NO: 3, and nucleotides 46-1536 of SEQ ID NO: 1.

Aspect 15: An expression vector comprising the nucleic acid according to any one of aspects 11 to 14.

Aspect 16: A host cell transformed with the expression vector according to aspect 15.

Aspect 17: A method of producing a recombinant protein having neuraminidase activity and/or β-galactoside-α2,6-sialyltransferase activity, wherein the method comprises the steps of:
1) transforming a host cell with an expression vector comprising the nucleic acid according to any one of Claims 11 to 14;
2) culturing the resulting transformed cell; and
3) isolating a protein having neuraminidase activity and/or β-galactoside-α2,6-sialyltransferase activity from the cultured transformed cell or the culture supernatant thereof.

Aspect 18: An antibody specifically recognizing the protein according to any one of aspects 1 to 10.

### ADVANTAGEOUS EFFECTS OF INVENTION

The present invention provides a novel protein having β-galactoside-α2,6-sialyltransferase activity and a nucleic acid encoding the protein and thereby contributes to provision of a means for synthesizing and producing sugar chains, which have been shown to have important functions *in vivo.* Sialic acid is often located at nonreducing termini of sugar chains of complex carbohydrates *in vivo* and is a very important sugar from the viewpoints of sugar chain functions. Accordingly, sialyltransferase is one of the most highly demanded enzymes among glycosyltransferases, and the provision of the novel sialyltransferase of the present invention meets such a high demand. The protein of the present invention also has neuraminidase activity that specifically cleaves sialic acid of α2,6-linkage. Neuraminidase that selectively cleaves sialic acid of α2,6-linkage has been found by the present inventors first.

### BRIEF DESCRIPTION OF DRAWINGS

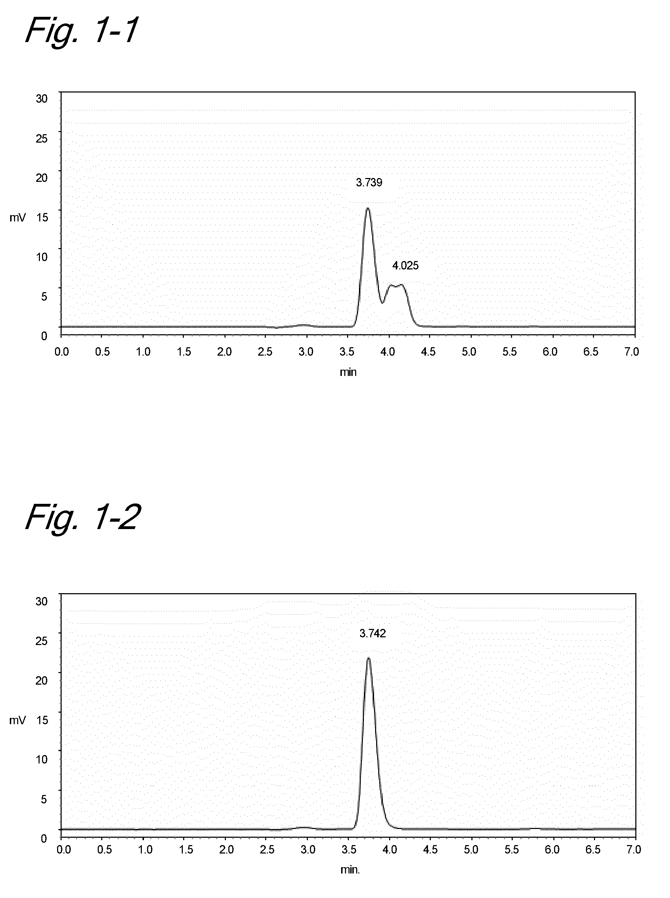

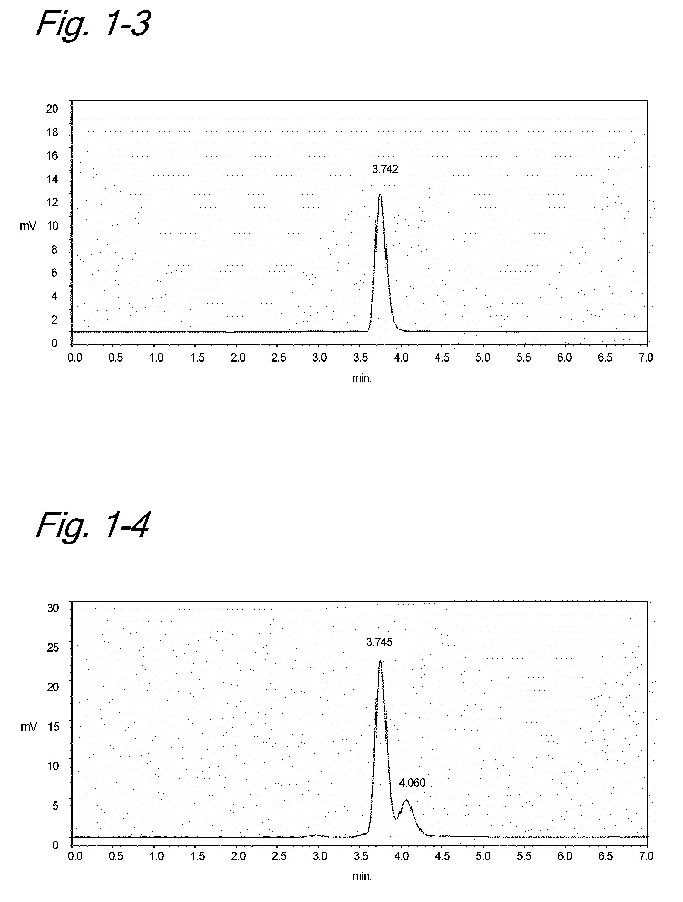

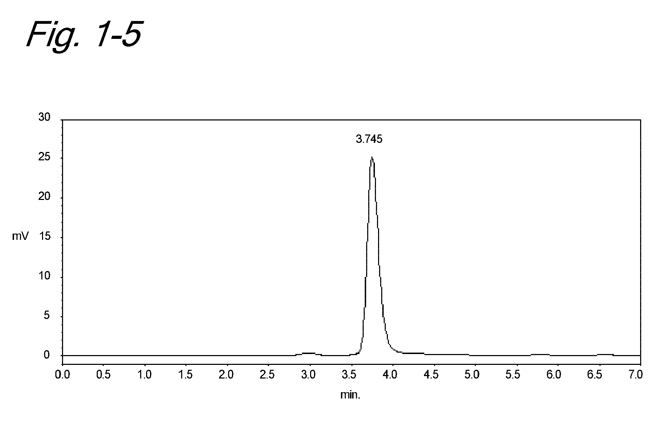

Fig. 1-1 is a graph showing the results of HPLC analysis of a reaction solution in which a crude enzyme solution prepared from cultured cells obtained by culturing *E. coli* cells transformed with an expression vector containing the β-galactoside-α2,6-sialyltransferase gene (SEQ ID NO: 3) derived from a JT-SHIZ-119 strain was reacted with pyridylaminated lactose (PA-lactose) and CMP-sialic acid. The peaks at retention times of 3.739 and 4.025 minutes represent PA-lactose and PA-6'-sialyllactose, respectively.
Fig. 1-2 is a graph showing the results of HPLC analysis in the case of mixing a crude enzyme solution prepared from cultured cells obtained by culturing *E. coli* cells transformed with an expression vector containing the β-galactoside-α2,6-sialyltransferase gene (SEQ ID NO: 3) derived from a JT-SHIZ-119 strain, with pyridylaminated lactose. This shows the results of a control experiment in which CMP-sialic acid serving as a sialic acid donor was not mixed into the reaction solution, relative to the experiment shown in Fig. 1-1. The peak at a retention time of 3.742 minutes represents PA-lactose.
Fig. 1-3 is a graph showing the results of HPLC analysis of a PA-lactose standard. The peak of PA-lactose appears at a retention time of 3.742 minutes.
Fig. 1-4 is a graph showing the results of HPLC analysis of a reaction solution obtained by reacting a known enzyme, β-galactoside-α2,6-sialyltransferase derived from a JT0160 strain, with PA-lactose and CMP-sialic acid (i.e., pyridylaminated α2,6-sialyllactose was produced). The peaks at retention times of 3.745 and 4.060 minutes represent PA-lactose and PA-6'-sialyllactose, respectively.
Fig. 1-5 is a graph showing the results of HPLC analysis of a reaction solution in which a known enzyme, α2,6-sialyltransferase derived from *Photobacterium damselae* strain JT0160, was reacted with PA-lactose. This is a control experiment in which CMP-sialic acid was not mixed into the reaction solution, relative to the experiment shown in Fig. 1-4. The peak at a retention time of 3.745 minutes represents PA-lactose.

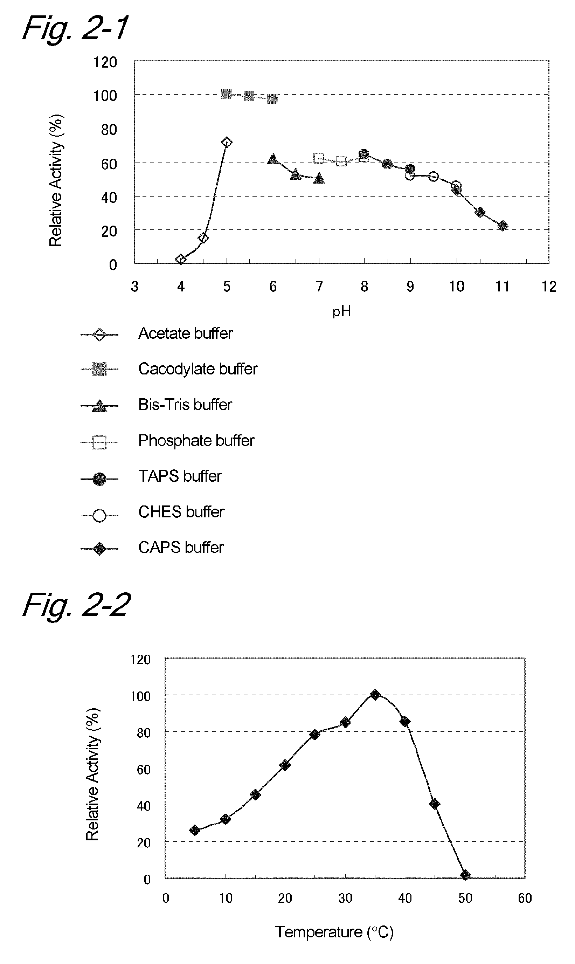

Fig. 2-1 is a graph showing the effect of reaction pH on the enzyme activity of recombinant β-galactoside-α2,6-sialyltransferase N1C0 (SEQ ID NO: 4) derived from a JT-SHIZ-119 strain. The types of buffers used and their pH ranges are as follows: acetate buffer (pH 4.0 to 5.0), cacodylate buffer (pH 5.0 to 6.0), Bis-Tris buffer (pH 6.0 to 7.0), phosphate buffer (pH 7.0 to 8.0), TAPS buffer (pH 8.0 to 9.0), CHES buffer (pH 9.0 to 10.0), and CAPS buffer (pH 10.0 to 11.0).
Fig. 2-2 is a graph showing the effect of reaction temperature on the enzyme activity of recombinant β-galactoside-α2,6-sialyltransferase N1C0 (SEQ ID NO: 4) derived from a JT-SHIZ-119 strain.

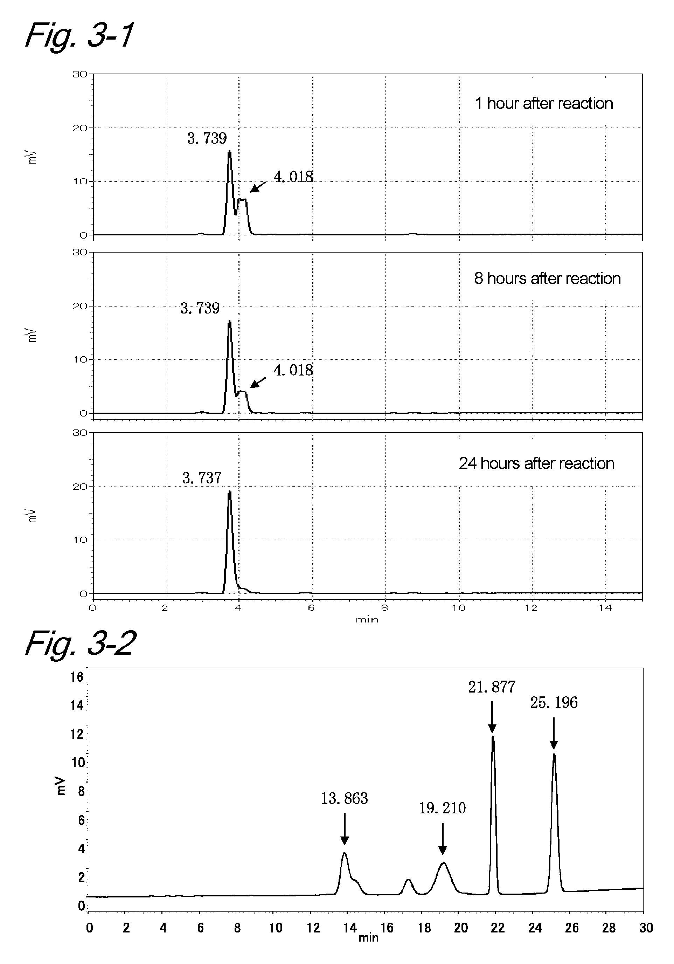

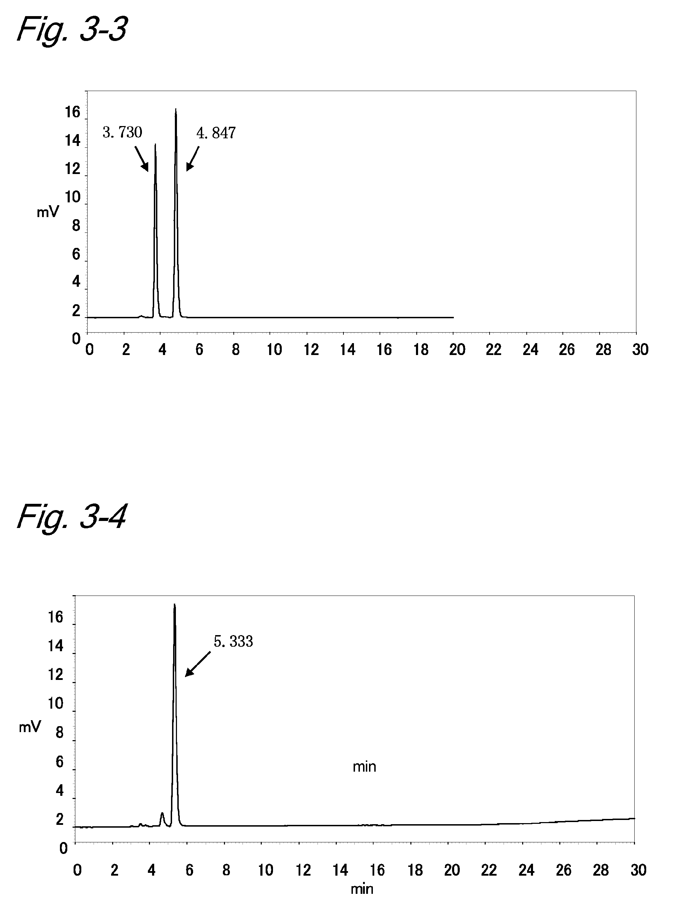

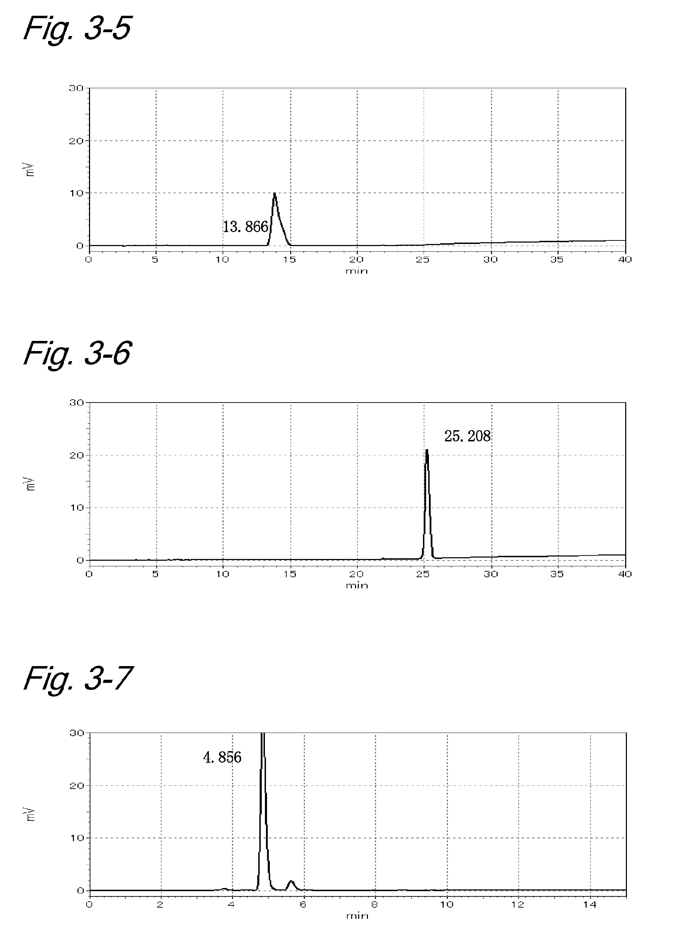

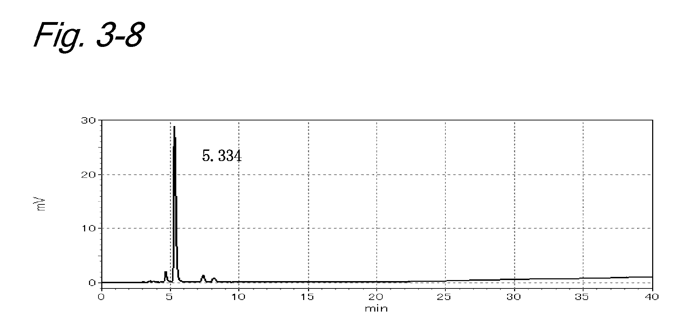

Fig. 3-1 includes graphs showing the results of HPLC analysis of a reaction solution in which a purified enzyme solution prepared from cultured cells obtained by culturing *E. coli* cells transformed with an expression vector containing the β-galactoside-α2,6-sialyltransferase gene (SEQ ID NO: 3) derived from a JT-SHIZ-119 strain was reacted with pyridylaminated lactose (PA-lactose) and CMP-sialic acid, and which was sampled at different points of time. The peaks at retention times of 3.737 to 3.739 and 4.018 minutes represent PA-lactose and PA-6'-sialyllactose, respectively.
Fig. 3-2 is a graph showing the results of HPLC analysis of PA-Sugar Chain 023, PA-Sugar Chain 022, and PA-Sugar Chain 021 standards in which the linkage mode of sialic acid is α2,6-linkage, and a sialic acid-free PA-Sugar Chain 001 standard (all of them are manufactured by Takara Bio Inc.). These are detected as peaks at retention times of 25.196, 19.210, 21.877, and 13.863 minutes, respectively.
Fig. 3-3 is a graph showing the results of HPLC analysis of a PA-Sugar Chain 029 standard in which the linkage mode of sialic acid is α2,3-linkage and a sialic acid-free PA-Sugar Chain 026 standard (all of them are manufactured by Takara Bio Inc.). These are detected as peaks at retention times of 4.847 and 3.730 minutes, respectively.
Fig. 3-4 is a graph showing the results of HPLC analysis of a PA-Sugar Chain 034 standard in which the linkage mode of sialic acid is α2,8-linkage (manufactured by Takara Bio Inc.). This is detected as a peak at a retention time of 5.333 minutes.
Fig. 3-5 is a graph showing the results of HPLC analysis of a reaction solution in which a purified enzyme solution prepared from cultured cells obtained by culturing *E. coli* cells transformed with an expression vector containing the β-galactoside-α2,6-sialyltransferase gene (SEQ ID NO: 3) derived from a JT-SHIZ-119 strain was reacted with PA-Sugar Chain 023. The peak at a retention time of 13.866 minutes corresponds to PA-Sugar Chain 001.
Fig. 3-6 is a graph showing the results of HPLC analysis when a PA-Sugar Chain 023 standard was reacted with a buffer and shows the results of a control experiment relative to the experiment shown in Fig. 3-3. The peak of PA-Sugar Chain 023 appears at a retention time of 25.208 minutes.
Fig. 3-7 is a graph showing the results of HPLC analysis of a reaction solution in which a purified enzyme solution prepared from cultured cells obtained by culturing *E. coli* cells transformed with an expression vector containing the β-galactoside-α2,6-sialyltransferase gene (SEQ ID NO: 3) derived from a JT-SHIZ-119 strain was reacted with PA-Sugar Chain 029. The peak at a retention time of 4.856 minutes corresponds to PA-Sugar Chain 029.
Fig. 3-8 is a graph showing the results of HPLC analysis of a reaction solution in which a purified enzyme solution prepared from cultured cells obtained by culturing *E. coli* cells transformed with an expression vector containing the β-galactoside-α2,6-sialyltransferase gene (SEQ ID NO: 3) derived from a JT-SHIZ-119 strain, was reacted with PA-Sugar Chain 034. The peak at a retention time of 5.334 minutes corresponds to PA-Sugar Chain 034.

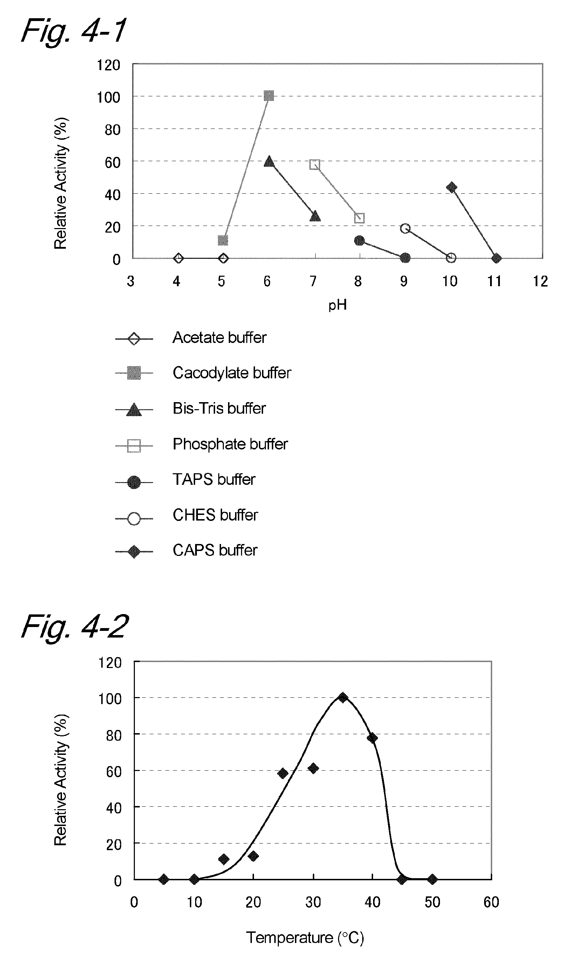

Fig. 4-1 is a graph showing the effect of reaction pH on the neuraminidase activity of recombinant β-galactoside-α2,6-sialyltransferase N1C0 (SEQ ID NO: 4) derived from a JT-SHIZ-119 strain. The types of buffers used and their pH ranges are as follows: acetate buffer (pH 4.0 to 5.0), cacodylate buffer (pH 5.0 to 6.0), Bis-Tris buffer (pH 6.0 to 7.0), phosphate buffer (pH 7.0 to 8.0), TAPS buffer (pH 8.0 to 9.0), CHES buffer (pH 9.0 to 10.0), and CAPS buffer (pH 10.0 to 11.0).
Fig. 4-2 is a graph showing the effect of reaction temperature on the neuraminidase activity of recombinant β-galactoside-α2,6-sialyltransferase N1C0 (SEQ ID NO: 4) derived from a JT-SHIZ-119 strain.

### DESCRIPTION OF EMBODIMENTS

The present invention will be described in further detail below.

### Definition

Unless otherwise specifically defined throughout the description, the scientific terms and technical terms used in relation to the present invention are intended to have the same meanings as those generally used by those skilled in the art.

The term "isolated" for molecules such as a protein, a nucleic acid, and an antibody throughout the description refers to a state of a molecule not substantially containing components present in its natural state. Examples of such a state include a state in which other molecules derived from species naturally producing the molecule are not substantially contained, a state in which the molecule is expressed in cells of species different from the species naturally producing the molecule or expressed in an established culture cell system, and a state in which the molecule is chemically synthesized. Furthermore, a molecule which is in an "isolated" state may be purified by any known process in the art so as not to contain substantially other components present in the natural state. Throughout the description, the term "not contain substantially" a component refers to a state in which the content of the component is reduced compared to that in the natural state. Examples of the state "not contain substantially" a component include a case in which the component is not contained at all, a case in which the component is contained in an amount below than the detection limit, and a case in which the content of the component is reduced to 1% or less, 5% or less, 10% or less, 25% or less, 50% or less, 75% or less, or 90% or less of that in the natural state.

The term "protein" throughout the description refers to a molecule containing at least two amino acid residues linked to each other by a peptide bond. The term "protein" throughout the description can also be referred to as "polypeptide."

The term "nucleic acid" throughout the description refers to a deoxyribonucleic acid (DNA) or ribonucleic acid (RNA) composed of at least two nucleotides. The term "nucleic acid" throughout the description is also referred to as "polynucleotide." It should be understood by those skilled in the art that in the case where a nucleic acid described by the nucleotide sequence of DNA is intended to be RNA, thymine in the nucleotide sequence of the DNA is replaced by uracil.

The term "β-galactoside-α2,6-sialyltransferase" throughout the description refers to a protein having an activity of transferring sialic acid from cytidine monophosphate (CMP)-sialic acid to the 6-position of a galactose residue in a sugar chain of a complex carbohydrate or a free sugar chain, to the 6-position of galactose present in an oligosaccharide such as lactose or *N*-acetyllactosamine, or to the 6-position of a monosaccharide, such as galactose, *N*-acetylgalactosamine, glucose, *N*-acetylglucosamine, or mannose, which can constitute a complex carbohydrate and has a hydroxyl group on the carbon at the 6-position. The term "β-galactoside-α2,6-sialyltransferase activity" throughout the description refers to such an activity for β-galactoside-α2,6-sialyltransferase described above.

The term "neuraminidase" throughout the description refers to a protein having an activity of cleaving sialic acid present at the nonreducing terminus of a sugar chain of a complex carbohydrate or a free sugar chain. The neuraminidase is also referred to as sialidase in this technical field. Three types of linkage modes between sialic acid and a sugar chain are known, i.e., α2,3-linkage, α2,6-linkage, and α2,8-linkage. Accordingly, neuraminidase may have an activity of cleaving at least one bond selected from the group consisting of α2,3-linkage, α2,6-linkage, and α2,8-linkage between sialic acid and a sugar chain. The term "neuraminidase activity" throughout the description is an activity for proteins and refers to an activity catalyzing a reaction of cleaving sialic acid present at the nonreducing terminus of a sugar chain of a complex carbohydrate or a free sugar chain, from the sugar chain. In a preferred embodiment, the protein of the present invention may have a neuraminidase activity selectively cleaving α2,6-linkage between sialic acid and a sugar chain.

The term "protein having neuraminidase activity and/or β-galactoside-α2,6-sialyltransferase activity" throughout the description may include a protein having either neuraminidase activity or β-galactoside-α2,6-sialyltransferase activity or a protein having both activities.

The term "sialic acid" throughout the description refers to a neuraminic acid derivative belonging to the sialic acid family. More specifically, it refers to, for example, *N*-acetylneuraminic acid (Neu5Ac), *N*-glycolylneuraminic acid (Neu5Gc), 5-deamino-5-hydroxyneuraminic acid (KDN), and disialic acid (i.e., di-*N*-acetylneuraminic acid: Neu5Acα2,8(9)Neu5Ac).

The "vector" throughout the description is a nucleic acid that can be used for introducing a nucleic acid linked thereto into a host cell. The "expression vector" refers to a vector that can induce expression of the protein encoded by the nucleic acid introduced by the vector. Examples of the vector include plasmid vectors and virus vectors.

The term "host cell" throughout the description refers to a cell that will be transfected or transformed with a vector. The host cell can be selected appropriately by those skilled in the art depending on the vector to be used. The host cell can be derived from a prokaryote such as *Escherichia coli* (*E. coli*) or a cell derived from a unicellular eukaryote such as yeast or a eukaryote such as a plant cell and an animal cell (e.g., human cell, monkey cell, hamster cell, rat cell, mouse cell, or insect cell).

### Protein

The present invention provides a novel protein having neuraminidase activity and/or β-galactoside-α2,6-sialyltransferase activity.

In an embodiment, the protein of the present invention is a protein comprising the amino acid sequence shown in SEQ ID NO: 2. The protein of the present invention may be a protein comprising the amino acid sequence shown in SEQ ID NO: 4. The amino acid sequence shown in SEQ ID NO: 4 is derived from the amino acid sequence shown in SEQ ID NO: 2 by removing amino acids 1 to 15 and adding methionine at the N-terminus. As described in Example 2 below, a protein (SHIZ119-N1C0) including the amino acid sequence shown in SEQ ID NO: 4 also retains the same β-galactoside-α2,6-sialyltransferase activity as a protein (SHIZ119-N0C0) including the amino acid sequence shown in SEQ ID NO: 2. This indicates that the presence of at least amino acids 16 to 511 of SEQ ID NO: 2 allows retention of β-galactoside-α2,6-sialyltransferase activity. For this reason, the protein of the present invention may be a protein including an amino acid sequence lacking all or part of amino acids 1 to 15 from amino acids 1 to 511 of SEQ ID NO: 2, or a protein comprising an amino acid sequence containing amino acids 16 to 511 of SEQ ID NO: 2.

In another embodiment, the protein of the present invention is a protein encoded by a nucleic acid including the nucleotide sequence shown in SEQ ID NO: 1. The protein of the present invention may be a protein encoded by a nucleic acid including the nucleotide sequence shown in SEQ ID NO: 3. The nucleotide sequence shown in SEQ ID NO: 3 corresponds to a sequence having an initiation codon (ATG) at the 5'-terminus of a nucleotide sequence containing nucleotides 46 to 1536 of SEQ ID NO: 1. The nucleotide sequences shown in SEQ ID NOs: 1 and 3 encode the amino acid sequences of SEQ ID NOs: 2 and 4, respectively. That is, the nucleotide sequences 46 to 1536 of SEQ ID NO: 1 encodes the amino acids 16 to 511 of SEQ ID NO: 2. Accordingly, the protein of the present invention may be a protein encoded by a nucleic acid comprising a nucleotide sequence containing nucleotides 46 to 1536 of SEQ ID NO: 1.

The present invention also encompasses mutants of the above-mentioned proteins of the present invention, i.e., mutant proteins having neuraminidase activity and/or β-galactoside-α2,6-sialyltransferase activity. Such mutant proteins also fall within the scope of the protein of the present invention.

The mutant protein of the present invention may be a protein including an amino acid sequence having deletion, substitution, insertion, and/or addition of one or more amino acids in an amino acid sequence selected from the group consisting of SEQ ID NO: 2, SEQ ID NO: 4, and amino acids 16 to 511 of SEQ ID NO: 2 and having neuraminidase activity and/or β-galactoside-α2,6-sialyltransferase activity. The substitution may be conservative substitution, which means the replacement of a certain amino acid residue by another residue having similar physicochemical characteristics. Non-limiting examples of the conservative substitution include replacement between aliphatic group-containing amino acid residues such as Ile, Val, Leu, and Ala and replacement between polar residues such as replacements between Lys and Arg; Glu and Asp; and Gln and Asn.

A mutant derived by deletion, substitution, insertion, and/or addition of amino acid or acids can be produced by subjecting a DNA encoding its wild-type protein to, for example, well-known site-directed mutagenesis (see, e.g., Nucleic Acid Research, Vol. 10, No. 20, pp. 6487-6500, 1982, which is hereby incorporated by reference in its entirety). Throughout the description, the term "one or more amino acids" indicates amino acids that can be deleted, substituted, inserted, and/or added by site-directed mutagenesis, and the number of the amino acids, which is nonlimiting, is preferably 20 or less, 15 or less, 10 or less, or 7 or less, and more preferably 5 or less.

Site-directed mutagenesis may be performed, for example, using a synthetic oligonucleotide primer that is complementary to single-stranded phage DNA to be mutated, except for having a specific mismatch, i.e., a desired mutation. That is, a complementary strand is synthesized by the phage using the synthetic oligonucleotide as a primer, and a host cell is transformed with the resulting double-stranded DNA. The transformed bacterial culture is plated on agar to form plaques from phage-containing single cells. As a result, in theory, 50% of new colonies contain phages with the mutation as a single strand, while the remaining 50% have the original sequence. At a temperature that allows hybridization with DNA completely identical to one having the above desired mutation, but not with DNA having the original strand, the resulting plaques are hybridized with a synthetic probe labeled by kinase treatment. Subsequently, plaques hybridized with the probe are picked up and cultured to collect the DNA.

The deletion, substitution, insertion, and/or addition of one or more amino acids in an amino acid sequence of a biologically active peptide, such as an enzyme, while retaining the activity, is performed by, as well as the site-directed mutagenesis, treating a gene with a mutagen or performing selective cleavage of a gene, then deletion, substitution, insertion, and/or addition of one or more selected nucleotides, and then ligation.

The mutant protein of the present invention may also be a protein encoded by a nucleic acid including a nucleotide sequence having deletion, substitution, insertion, and/or addition of one or more nucleotides in a nucleotide sequence selected from the group consisting of SEQ ID NO: 1, SEQ ID NO: 3, and nucleotides 46-1536 of SEQ ID NO: 1 and having neuraminidase activity and/or β-galactoside-α2,6-sialyltransferase activity. The deletion, substitution, insertion, and/or addition of a nucleotide or nucleotides may be performed by site-directed mutagenesis or another method described above.

Furthermore, the mutant protein of the present invention may be a protein including an amino acid sequence having an amino acid identity of at least 95%, preferably 97% or more, 98% or more, 98.5% or more, 99% or more, or 99.5% or more, and more preferably 99.8% or more with an amino acid sequence selected from the group consisting of SEQ ID NO: 2, SEQ ID NO: 4, and amino acids 16 to 511 of SEQ ID NO: 2 and having neuraminidase activity and/or β-galactoside-α2,6-sialyltransferase activity.

Alternatively, the mutant protein of the present invention may be a protein encoded by a nucleic acid having an identity of at least 95%, preferably 97% or more, 98% or more, 98.5% or more, 99% or more, or 99.5% or more, and more preferably 99.8% or more with a nucleotide sequence selected from the group consisting of SEQ ID NO: 1, SEQ ID NO: 3, and nucleotides 46-1536 of SEQ ID NO: 1 and having neuraminidase activity and/or β-galactoside-α2,6-sialyltransferase activity.

The percent identity between two amino acid sequences may be determined by visual inspection and mathematical calculation. Alternatively, the percent identity between two protein sequences may be determined by comparing sequence information based on the algorithm of Needleman, S. B. and Wunsch, C. D. (J. Mol. Biol., 48: 443-453, 1970) and using the GAP computer program available from the University of Wisconsin Genetics Computer Group (UWGCG). The preferred default parameters for the GAP program include: (1) a scoring matrix, blosum62, as described by Henikoff, S. and Henikoff, J. G. (Proc. Natl. Acad. Sci. USA, 89:10915-10919, 1992); (2) a gap weight of 12; (3) a gap length weight of 4; and (4) no penalty for end gaps.

Other programs for sequence comparison used by those skilled in the art may also be used. The percent identity can be determined by comparing sequence information using, for example, the BLAST program described by Altschul, et al. (Nucl. Acids. Res., 25, pp. 3389-3402, 1997). This program is available from the web sites of the National Center for Biotechnology Information (NCBI) or the DNA Data Bank of Japan (DDBJ) on the Internet. The details of various conditions (parameters) for identity search using the BLAST program are shown on these web sites, and default values are commonly used for search although a part of the settings may be partially changed as appropriate. Alternatively, the percent identity between two amino acid sequences may be determined using a program such as genetic information processing software GENETYX (Genetyx Corporation, Japan) or using an algorithm such as FASTA. In such a case, default values may be used to conduct a search.

The percent identity between two nucleic acid sequences can be determined by visual inspection and mathematical calculation. More preferably, the comparison is performed by comparing sequence information using a computer program. A typical preferred computer program is the Genetic Computer Group (GCG; Madison, WI) Wisconsin package version 10.0 program, "GAP" (Devereux, et al., 1984, Nucl. Acids Res., 12: 387). This "GAP" program can be used not only for comparison between two nucleic acid sequences but also for comparison between two amino acid sequences and comparison between a nucleic acid sequence and an amino acid sequence. The preferred default parameters for the "GAP" program include: (1) the GCG implementation of a unary comparison matrix (containing a value of 1 for identities and 0 for non-identities) for nucleotides, and the weighted amino acid comparison matrix of Gribskov and Burgess, Nucl. Acids Res., 14: 6745, 1986, as described in Schwartz and Dayhoff, eds., "Atlas of Polypeptide Sequence and Structure," National Biomedical Research Foundation, pp. 353-358, 1979, or other comparable comparison matrices; (2) a penalty of 30 for each gap for amino acids and an additional penalty of 1 for each symbol in each gap, or a penalty of 50 for each gap for nucleotide sequences and an additional penalty of 3 for each symbol in each gap; (3) no penalty for end gaps; and (4) no maximum penalty for long gaps. Other sequence comparison programs used by those skilled in the art can also be used. For example, the BLASTN program version 2.2.7, which is available via the National Library of Medicine website: http://www.ncbi.nlm.nih.gov/blast/bl2seq/bls.html, or the UW-BLAST 2.0 algorithm can be used. Setting of the standard default parameters for the UW-BLAST 2.0 is described at the following Internet site: http://blast.wustl.edu. In addition, the BLAST algorithm uses the BLOSUM62 amino acid scoring matrix, and optional parameters that can be used are as follows: (A) inclusion of a filter to mask segments of the query sequence having low compositional complexity (determined by the SEG program of Wootton and Federhen (Computers and Chemistry, 1993); also see Wootton and Federhen, 1996, "Analysis of compositionally biased regions in sequence databases," Methods Enzymol., 266: 544-71) or segments consisting of short-periodicity internal repeats (determined by the XNU program of Claverie and States (Computers and Chemistry, 1993)), and (B) a statistical significance threshold for reporting matches against database sequences or E-score (the expected probability of matches being found merely by chance, in accordance with the statistical model (Karlin and Altschul, 1990); if the statistical significance ascribed to a match is greater than the E-score threshold, the match will not be reported.); preferred E-score threshold values are 0.5, or in order of increasing preference, 0.25, 0.1, 0.05, 0.01, 0.001, 0.0001, le-5, le-10, le-15, le-20, le-25, le-30, le-40, le-50, le-75, or le-100.

The mutant protein of the present invention may also be a protein encoded by a nucleic acid including a nucleotide sequence hybridizable under stringent conditions with the complementary strand of a nucleotide sequence selected from the group consisting of SEQ ID NO: 1, SEQ ID NO: 3, and nucleotides 46-1536 of SEQ ID NO: 1 and having neuraminidase activity and/or β-galactoside-α2,6-sialyltransferase activity.

Herein, the term "under stringent conditions" refers to hybridization that occurs under moderately or highly stringent conditions. Specifically, moderately stringent conditions readily can be determined by those having ordinary skill in the art, e.g., on the basis of the length of DNA. The basic conditions are set forth by Sambrook, et al., Molecular Cloning: A Laboratory Manual, 3rd edition, chapters 6 and 7, Cold Spring Harbor Laboratory Press, 2001 and include the use of a prewashing solution for nitrocellulose filters 5 × SSC, 0.5% SDS, and 1.0 mM EDTA (pH 8.0), hybridization conditions of about 50% formamide, 2 × SSC to 6 × SSC at about 40°C to 50°C (or other similar hybridization solutions, such as Stark's solution, in about 50% formamide at about 42°C), and washing conditions of, for example, about 40°C to 60°C, 0.5 to 6 × SSC, and 0.1% SDS. Preferably, moderately stringent conditions include hybridization conditions (and washing conditions) at about 50°C and 6 × SSC. Highly stringent conditions can also be readily determined by those skilled in the art, for example, depending on the length of DNA.

In general, highly stringent conditions include hybridization and/or washing at higher temperature and/or lower salt concentration (for example, hybridization at about 65°C, 6 × SSC to 0.2 × SSC, preferably 6 × SSC, more preferably 2 × SSC, most preferably 0.2 × SSC), compared to the moderately stringent conditions, and also include the hybridization conditions defined above with washing at approximately 65°C to 68°C, 0.2 × SSC, and 0.1% SDS. With the hybridization and washing buffer, SSPE (1 × SSPE is 0.15 M NaCl, 10 mM NaH₂PO₄, and 1.25 mM EDTA, pH 7.4) can be substituted for SSC (1 × SSC is 0.15 M NaCl and 15 mM sodium citrate). The washing is performed for 15 minutes after completion of the hybridization.

A commercially available hybridization kit including a probe that is not a radioactive substance can also be used. Specifically, hybridization utilizing an ECL direct labeling & detection system (manufactured by Amersham) is available. For example, stringent hybridization is performed using the hybridization buffer included in the kit to which a blocking reagent and NaCl are added in concentrations of 5% (w/v) and 0.5 M, respectively, under the following conditions: at 42°C for 4 hours and washing twice in 0.4% SDS, 0.5 × SSC at 55°C for 20 minutes and once in 2 × SSC at room temperature for 5 minutes.

The sialyltransferase activity may be measured by a known method, for example, the process described in J. Biochem., 120, 104-110 (1996) (which is hereby incorporated by reference in its entirety). For example, the enzyme activity can be evaluated by performing an enzyme reaction using CMP-NeuAc (*N*-acetylneuraminic acid) as a sugar donor substrate and lactose as a sugar acceptor substrate and evaluating the amount of the reaction product, i.e., sialyllactose. Note that one enzyme unit (1 U) of sialyltransferase is defined as the amount of the enzyme required to transfer one micromole of sialic acid per minute.

The linkage mode of sialic acid transferred to the sugar acceptor substrate can be determined by, but is not limited to, any procedure known to those skilled in the art, for example, a method using a pyridylaminated sugar chain or nuclear magnetic resonance spectroscopy (NMR) of the reaction product. The method using a pyridylaminated sugar chain involves an enzyme reaction using the pyridylaminated sugar chain as the sugar acceptor substrate. More specifically, an enzyme reaction is performed using pyridylaminated lactose (Galβ1-4Glc-PA, manufactured by Takara Bio Inc.) as a sugar acceptor substrate and CMP-NeuAc as a sugar donor substrate, and the reaction product is analyzed by high performance liquid chromatography (HPLC). From the retention time of the reaction product, the position at which sialic acid was transferred is determined.

The neuraminidase activity may be measured by a known method. For example, the enzyme activity can be evaluated through hydrolysis of sialic acid under the effect of neuraminidase on a sialic acid-containing sugar chain and determining the amount of the reaction product; i.e., the amount of the sugar chain from which sialic acid was released or the amount of free sialic acid. Note that one enzyme unit (1 U) of neuraminidase is defined as the amount of the enzyme required to release one micromole of sialic acid per minute.

The substrate specificity of neuraminidase, that is, the linkage mode of sialic acid to a sugar chain that is cleaved by the neuraminidase can be determined by, but not limited to, a method using a pyridylaminated sugar chain. More specifically, the enzyme reaction by neuraminidase is performed using PA sugar chains in which sialic acid is linked by α2,3-linkage, α2,6-linkage, or α2,8-linkage (for example, pyridylaminated sugar chains such as PA-Sugar Chain 029, PA-Sugar Chain 023, and PA-Sugar Chain 034 available from Takara Bio Inc.). The reaction product is analyzed by high performance liquid chromatography, and the amount of sialic acid that was cleaved is calculated from the retention time and peak area of the reaction product.

In an embodiment of the present invention, the protein of the present invention is derived from microorganisms belonging to the genus Photobacterium. The protein of the present invention may be derived from any microorganism belonging to the genus Photobacterium or may be a protein derived from a new species of microorganism belonging to the genus Photobacterium. In a preferred embodiment, the protein of the present invention is derived from a microorganism belonging to *Photobacterium leiognathi.*

The protein of the present invention may be characterized by any one of the following nonlimiting enzymological properties and physicochemical properties: The optimum pH for the β-galactoside-α2,6-sialyltransferase activity of the protein of the present invention is in the range of, but is not limited to, pH 4.0 to 9.0, preferably pH 5.0 to 9.0, pH 5.0 to 8.0, pH 4.0 to 8.0, pH 4.0 to 6.0, pH 4.5 to 6.0, or pH 5.0 to 6.0, and more preferably pH 5.0. The optimum temperature for the β-galactoside-α2,6-sialyltransferase activity of the protein of the present invention is in the range of, but is not limited to,30°C to 40°C, preferably 35°C to 40°C or 35°C to 38°C , and more preferably 35°C. The optimum pH for the neuraminidase activity of the protein of the present invention is in the range of, but is not limited to, pH 5.0 to 7.0, preferably pH 6.0 to 7.0, and more preferably pH 6.0. The optimum temperature for the neuraminidase activity of the protein of the present invention is in the range of, but not limited to, 25°C to 40°C, preferably 30°C to 40°C, and more preferably 35°C. The protein of the present invention has a molecular weight of about 50000±5000 Da, as measured by SDS-PAGE analysis.

The protein of the present invention may be a protein shown below:
(1) a protein including an amino acid sequence having deletion, substitution, insertion, and/or addition of one or more amino acids in an amino acid sequence selected from the group consisting of SEQ ID NO: 2, amino acids 16 to 511 of SEQ ID NO: 2, and SEQ ID NO: 4;
(2) a protein including an amino acid sequence having an amino acid identity of at least 97% with an amino acid sequence selected from the group consisting of SEQ ID NO: 2, amino acids 16 to 511 of SEQ ID NO: 2, and SEQ ID NO: 4;
(3) a protein including an amino acid sequence encoded by a nucleic acid having an identity of at least 97% with a nucleotide sequence selected from the group consisting of SEQ ID NO: 1, nucleotides 46-1536 of SEQ ID NO: 1, and SEQ ID NO: 3; or
(4) a protein encoded by a nucleic acid including a nucleotide sequence hybridizable under stringent conditions with the complementary strand of a nucleotide sequence selected from the group consisting of SEQ ID NO: 1, nucleotides 46-1536 of SEQ ID NO: 1, and SEQ ID NO: 3.

While the protein of the present invention has neuraminidase activity and/or β-galactoside-α2,6-sialyltransferase activity, and the specific activity of the sialyltransferase activity is larger than that of the neuraminidase activity. Thus, in a reaction system containing a CMP-sialic acid, it functions as a sialyltransferase and forms a sugar chain to which sialic acid has bound.

In a reaction system not containing CMP-sialic acid, the sialyltransferase activity does not function. Accordingly, the protein of the present invention functions as neuraminidase only to hydrolyze sialic acid from a sialic acid-binding sugar chain.

### Nucleic acid

The present invention provides a nucleic acid encoding a protein having neuraminidase activity and/or β-galactoside-α2,6-sialyltransferase activity.

In an embodiment, the nucleic acid of the present invention is a nucleic acid encoding a protein including an amino acid sequence selected from the group consisting of SEQ ID NO: 2, SEQ ID NO: 4 (a sequence derived from the amino acid sequence shown in SEQ ID NO: 2 by removing amino acids 1 to 15 and adding methionine at the N-terminus), and amino acids 16 to 511 of SEQ ID NO: 2. The nucleic acid of the present invention may be a nucleic acid including a nucleotide sequence selected from the group consisting of SEQ ID NO: 1, SEQ ID NO: 3 (a sequence having an initiation codon (ATG) at the 5'-terminus of the nucleotides 46-1536 of SEQ ID NO: 1), and nucleotides 46 to 1536 of SEQ ID NO: 1.

The nucleic acid of the present invention may be a mutant of the above-mentioned nucleic acid; i.e., a mutant nucleic acid encoding a protein having neuraminidase activity and/or β-galactoside-α2,6-sialyltransferase activity. Such a nucleic acid mutant also falls within the scope of the present invention.

Such a nucleic acid mutant is a nucleic acid encoding a protein comprising an amino acid sequence having deletion, substitution, insertion, and/or addition of one or more amino acids in an amino acid sequence selected from the group consisting of SEQ ID NO: 2, SEQ ID NO: 4, and amino acids 16 to 511 of SEQ ID NO: 2 and having neuraminidase activity and/or β-galactoside-α2,6-sialyltransferase. The nucleic acid mutant of the present invention is also a nucleic acid comprising a nucleotide sequence having deletion, substitution, insertion, and/or addition of one or more nucleotides in a nucleotide sequence selected from the group consisting of SEQ ID NO: 1, SEQ ID NO: 3, and nucleotides 46 to 1536 of SEQ ID NO: 1. The deletion, substitution, insertion, and/or addition of amino acid or nucleotide can be introduced as described above.

Alternatively, such a nucleic acid mutant is a nucleic acid encoding a protein comprising an amino acid sequence having an identity of at least 95%, preferably 97% or more, 98% or more, 98.5% or more, 99% or more, or 99.5% or more, and more preferably 99.8% or more with an amino acid sequence selected from the group consisting of SEQ ID NO: 2, SEQ ID NO: 4, and amino acids 16 to 511 of SEQ ID NO: 2 and having neuraminidase activity and/or β-galactoside-α2,6-sialyltransferase activity. The nucleic acid mutant of the present invention is also a nucleic acid having an identity of at least 95%, preferably 97% or more, 98% or more, 98.5% or more, 99% or more, or 99.5% or more, and more preferably 99.8% or more with a nucleotide sequence selected from the group consisting of SEQ ID NO: 1, SEQ ID NO: 3, and nucleotides 46-1536 of SEQ ID NO: 1 and encoding a protein having neuraminidase activity and/or β-galactoside-α2,6-sialyltransferase activity. The identity between amino acid sequences or nucleotide sequences can be determined as described above.

Furthermore, such a nucleic acid mutant may be a nucleic acid including a nucleotide sequence hybridizable under stringent conditions or highly stringent conditions with the complementary strand of a nucleotide sequence selected from the group consisting of SEQ ID NO: 1, SEQ ID NO: 3, and nucleotides 46-1536 of SEQ ID NO: 1 and encoding a protein having neuraminidase activity and/or β-galactoside-α2,6-sialyltransferase activity. The stringent conditions or highly stringent conditions are as defined above.

The nucleic acid of the present invention may be a nucleic acid shown below:
(1) a nucleic acid encoding a protein comprising an amino acid sequence having deletion, substitution, insertion, and/or addition of one or more amino acids in an amino acid sequence selected from the group consisting of SEQ ID NO: 2, amino acids 16 to 511 of SEQ ID NO: 2, and SEQ ID NO: 4;
(2) a nucleic acid encoding a protein comprising an amino acid sequence having an amino acid identity of at least 97% with an amino acid sequence selected from the group consisting of SEQ ID NO: 2, amino acids 16 to 511 of SEQ ID NO: 2, and SEQ ID NO: 4;
(3) a nucleic acid having an identity of at least 97% with a nucleotide sequence selected from the group consisting of SEQ ID NO: 1, nucleotides 46-1536 of SEQ ID NO: 1, and SEQ ID NO: 3; or
(4) a nucleic acid including a nucleotide sequence hybridizable under stringent conditions with the complementary strand of a nucleotide sequence selected from the group consisting of SEQ ID NO: 1, nucleotides 46-1536 of SEQ ID NO: 1, and SEQ ID NO: 3.

### Microorganism expressing the protein of the present invention

The present inventors have found that microorganisms belonging to the genus Photobacterium of the family Vibrionaceae express a novel β-galactoside-α2,6-sialyltransferase and that the enzyme also has neuraminidase activity. Thus, the present invention provides a microorganism expressing the protein having neuraminidase activity and/or β-galactoside-α2,6-sialyltransferase activity. The microorganism of the present invention belongs to the genus Photobacterium and has an ability of producing the protein having neuraminidase activity and/or β-galactoside-α2,6-sialyltransferase activity. An example of the microorganism having the ability of producing the protein having neuraminidase activity and/or β-galactoside-α2,6-sialyltransferase activity and belonging to the genus Photobacterium is *Photobacterium leiognathi* strain JT-SHIZ-119. Note that the microorganisms of the genus Photobacterium are generally marine bacteria, which are separated from sea water, or marine fish or shellfish.

The microorganism of the present invention can be separated, for example, through the following screening procedures. Sea water, sea sand, sea mud, or marine fish or shellfish is used as a microorganism source. Sea water, sea sand, and sea mud may be used directly or diluted with sterilized sea water for use as an inoculum. In the case of marine fish and shellfish, their surface mucus or the like is collected by scrubbing with a loop and is then used as an inoculum; or their internal organs are homogenized in sterilized sea water, and the resulting fluid is used as an inoculum. Such an inoculum is applied onto a plate medium such as marine broth agar 2216 medium (Becton, Dickinson and Company) or sodium chloride-supplemented nutrient agar medium (Becton, Dickinson and Company) to obtain marine microorganisms growing under various temperature conditions. After the resulting microorganisms have been pure-cultured in a usual manner, each microorganism is cultured using a liquid medium such as marine broth 2216 medium (Becton, Dickinson and Company) or sodium chloride-supplemented nutrient broth medium (Becton, Dickinson and Company). After the microorganisms are fully grown, the cells are collected by centrifugation from each culture solution. To the collected cells, 20 mM cacodylate buffer (pH 6.0) containing 0.2% Triton X-100 (Kanto Chemical Co., Ltd.), a surfactant, is added, and the cells are suspended therein. This cell suspension is ultrasonicated under ice cooling to homogenize the cells. This cell homogenate is used as an enzyme solution and measured for its sialyltransferase activity in a usual manner, to thereby obtain a strain having sialyltransferase activity.

The *Photobacterium sp.* strain JT-SHIZ-119 that produces β-galactoside-α2,6-sialyltransferase characterized by having a reaction temperature of 35°C to 40°C described in the present invention was obtained by the above-mentioned screening.

### Method of producing the protein of the present invention

The present invention also relates to a method of producing a protein having neuraminidase activity and/or β-galactoside-α2,6-sialyltransferase activity. In a preferred embodiment, the method of the present invention produces the protein of the present invention.

### (1) Method of producing recombinant protein

The present invention provides an expression vector containing a nucleic acid of the present invention and a host cell containing the expression vector. Moreover, the present invention also provides a method of producing a recombinant protein having neuraminidase activity and/or β-galactoside-α2,6-sialyltransferase activity by culturing a host cell containing the expression vector under conditions suitable for expressing the recombinant protein and collecting the expressed recombinant protein.

To produce the recombinant protein of the present invention, an expression vector chosen depending on the host to be used is inserted with a nucleic acid sequence encoding a protein having neuraminidase activity and/or β-galactoside-α2,6-sialyltransferase and being operably linked to a suitable transcription or translation regulatory nucleotide sequence derived from a gene of mammalian, microorganism, viral, insect, or other origin. Examples of the regulatory sequence include a transcription promoter, an operator, an enhancer, an mRNA ribosome binding site, and suitable sequences regulating the initiation and termination of transcription and translation.

The nucleic acid sequence encoding the protein having neuraminidase activity and/or β-galactoside-α2,6-sialyltransferase activity to be inserted into the vector of the present invention is a nucleotide sequence of the above-described nucleic acid of the present invention. The sequence may include a leader sequence or may not include the same. If the nucleotide sequence includes a leader sequence, the leader sequence may correspond to nucleotides 1 to 42 of SEQ ID NO: 1 or may be replaced by a leader sequence derived from another organism. An expression system can be designed such that the expressed protein is secreted to the outside of the host cells by replacing the leader sequence.

The recombinant protein having neuraminidase activity and/or β-galactoside-α2,6-sialyltransferase activity according to the present invention can also be expressed as a fusion protein by inserting into a vector first a nucleic acid encoding the protein and subsequently a nucleic acid linked to a nucleic acid encoding a His tag, a FLAG^{™} tag (tag including an amino acid sequence: DYKDDDDK (SEQ ID NO: 17)), glutathione-S-transferase or the like. The enzyme of the present invention readily can be purified and detected by expressing the enzyme as such a fusion protein.

Examples of a host cell suitable for expressing the protein of the present invention include prokaryotic cells, yeast, and higher eukaryotic cells. Examples of cloning and expression vectors suitable for use in bacterial, fungal, yeast, and mammalian host cells are described, for example, in Pouwels, et al., Cloning Vectors: A Laboratory Manual, Elsevier, New York, (1985) (which is hereby incorporated by reference in its entirety).

The prokaryotes include Gram-negative and Gram-positive bacteria such as *E. coli* or *Bacillus subtilis.* For a prokaryotic cell such as *E. coli* used as a host, the protein of the present invention may be designed to have an N-terminal methionine residue for the purpose of facilitating the expression of a recombinant polypeptide within prokaryotic cells. This N-terminal methionine can be cleaved from the expressed recombinant protein.

Expression vectors to be used in prokaryotic host cells generally contain one or more phenotype selectable marker genes. Such a phenotype selectable marker gene is, for example, a gene imparting antibiotic resistance or autotrophy. Examples of expression vectors suitable for prokaryotic host cells include commercially available plasmids such as pBR322 (ATCC37017) or derivatives thereof. The pBR322 contains genes for ampicillin and tetracycline resistance, and thereby transformed cells easily can be identified. DNA sequences of a suitable promoter and a nucleic acid encoding β-galactoside-α2,6-sialyltransferase are inserted into this pBR322 vector. Other examples of commercially available vectors include pKK223-3 (Pharmacia Fine Chemicals, Inc. Uppsala, Sweden) and pGEM1 (Promega Biotech AB, Madison, Wisconsin, United States).

Examples of promoter sequences usually used in expression vectors for prokaryotic host cells include tac promoters, β-lactamase (penicillinase) promoters, and lactose promoters (Chang, et al., Nature 275: 615, 1978; and Goeddel, et al., Nature 281: 544, 1979, which are hereby incorporated by reference in their entirety).

Alternatively, the recombinant protein of the present invention may be expressed in yeast host cells. *Saccharomyces* (e.g., *S. cerevisiae*) is preferably used, but other genera of yeast, such as *Pichia* or *Kluyveromyces,* may also be used. Yeast vectors often contain a sequence of replication origin from a 2µ yeast plasmid, an autonomously replicating sequence (ARS), a promoter region, a sequence for polyadenylation, a sequence for transcription termination, and a selectable marker gene. A yeast α-factor leader sequence can also be used to induce secretion of a recombinant β-galactoside-α2,6-sialyltransferase protein. There are also known other leader sequences that are suitable for facilitating recombinant polypeptide secretion from yeast hosts. A method of transforming yeast is described, for example, in Hinnen, et al., Proc. Natl. Acad. Sci. USA, 75: 1929-1933, 1978 (which is hereby incorporated by reference in its entirety).

The recombinant protein of the present invention can also be expressed using a mammalian or insect host cell culture system. Established cell lines of mammalian origin can also be used. Transcription and translation control sequences for mammalian host cell expression vectors can be obtained from viral genomes. Promoter and enhancer sequences usually used are derived from, for example, polyoma virus or adenovirus 2. Other gene elements for expressing structural gene sequences in mammalian host cells may also be provided by using DNA sequences derived from the SV40 viral genome, e.g., SV40 origin, early and late promoters, enhancers, splice sites, and polyadenylation sites. Vectors for use in mammalian host cells can be constructed by, for example, the method of Okayama and Berg (Mol. Cell. Biol., 3: 280, 1983, which is hereby incorporated by reference in its entirety).

One method of producing a protein having neuraminidase activity and/or β-galactoside-α2,6-sialyltransferase activity according to the present invention includes culturing host cells transformed with an expression vector containing a nucleic acid sequence encoding the protein under conditions allowing expression of the protein. Then, the protein is collected from the culture medium or cell extract in a manner suitable for the expression system used.

The procedure for purifying a recombinant protein having neuraminidase activity and/or β-galactoside-α2,6-sialyltransferase activity is selected appropriately depending on such factors as what type of host is used and whether the protein of the present invention is secreted into the culture medium. Examples of the procedure for purifying a recombinant protein include column chromatographic approaches using, for example, an anion exchange column, a cation exchange column, a gel filtration column, a hydroxyapatite column, a CDP-hexanolamine agarose column, a CMP-hexanolamine agarose column, or a hydrophobic column; Native-PAGE; and combinations thereof. Alternatively, in the case of expressing the recombinant protein in a form fused with a tag or the like for facilitating purification, affinity chromatography may be used for purification. For example, in the case of fusion protein with a histidine tag, a FLAG^{™} tag, or glutathione-S-transferase (GST), purification can be accomplished by affinity chromatography using a nitrilotriacetic acid (Ni-NTA) column, an anti-FLAG antibody-bound column, or a glutathione-bound column, respectively.

Although the recombinant protein having neuraminidase activity and/or β-galactoside-α2,6-sialyltransferase activity may be purified to give an electrophoretically single band, the β-galactoside-2,6-sialyltransferase of the present invention may be of a completely purified or partially purified form because it has sufficient activity even in a partially purified form.

### Antibody

The present invention provides an antibody against the protein having neuraminidase activity and/or β-galactoside-α2,6-sialyltransferase activity according to the present invention. The antibody of the present invention may be prepared against the protein of the present invention or a fragment thereof. The fragment of the protein of the present invention has a sequence including at least 6 amino acids, at least 10 amino acids, at least 20 amino acids, or at least 30 amino acids in the amino acid sequence of the enzyme.

The antibody may be prepared by immunizing an animal used in the art for preparing an antibody; which is, for example, but is not limited to, a mouse, a rat, a rabbit, a guinea pig, or a goat, with the protein of the present invention or a fragment thereof. The antibody may be either polyclonal or monoclonal. The antibody can be prepared based on an antibody-producing process well known to those skilled in the art.

The fragments of the antibody of the present invention also falls within the scope of the present invention. Examples of the fragments of the antibody include Fab, F(ab')₂, Fv, and fragments containing complementarity determining region (CDR).

The antibody of the present invention can be used for collecting the protein having neuraminidase activity and/or β-galactoside-α2,6-sialyltransferase activity according to the present invention by affinity purification. The antibody of the present invention can also be used for detecting the protein of the present invention in assays such as western blotting and ELISA.

### EXAMPLES

The present invention will now be described in more detail with reference to examples below, which are not intended to limit the technical scope of the invention. Based on the description in the specification, modifications and changes will be apparent to those skilled in the art, and such modifications and changes fall within the technical scope of the invention.

### Example 1: Screening and strain identification of microorganisms producing β-galactoside-α2,6-sialyltransferase

### (1) Screening

Sea water, sea sand, sea mud, or marine fish or shellfish was used as an inoculum. This inoculum was applied onto a plate medium containing marine broth agar 2216 medium (Becton, Dickinson and Company) to obtain microorganisms growing at 15°C, 25°C, or 30°C. After the resulting microorganisms were pure-cultured in a usual manner, each microorganism was cultured using a liquid medium containing marine broth 2216 medium (Becton, Dickinson and Company). After the microorganisms were sufficiently grown, the cells were collected from each culture solution by centrifugation. To the collected cells, 20 mM cacodylate buffer (pH 6.0) containing 0.2% Triton X-100 (Kanto Chemical Co., Ltd.) was added, and the cells were suspended therein. This cell suspension was ultrasonicated under ice cooling to homogenize the cells. This cell homogenate solution was used as a crude enzyme solution, and the sialyltransferase activity thereof was measured for selecting a strain having sialyltransferase activity, i.e., a JT-SHIZ-119 strain.

Sialyltransferase activity was measured by a method described in J. Biochem., 120, 104-110 (1996) (which is hereby incorporated by reference in its entirety). Specifically, the enzyme reaction was performed using a reaction solution (30 µL) containing CMP-NeuAc (70 nmol, containing about 20000 cpm CMP-NeuAc in which NeuAc was labeled with ¹⁴C; NeuAc represents *N*-acetylneuraminic acid) as a sugar donor substrate, lactose (1.25 µmol) as a sugar acceptor substrate, NaCl added to give a concentration of 0.5 M, and the enzyme prepared as described above. The enzyme reaction was carried out at 25°C for about 10 to 180 minutes. After completion of the reaction, 1.97 mL of 5 mM phosphate buffer (pH 6.8) was added to the reaction solution, which was then applied to a Dowex 1 × 8 (PO₄³⁻ form, 0.2 × 2 cm, manufactured by Bio-Rad Laboratories, Inc.) column. The radioactivity contained in the reaction product contained in the eluate (0 to 2 mL) from this column, that is, sialyllactose, was measured to calculate the enzyme activity. One enzyme unit (1 U) is defined as the amount of enzyme required to transfer one micromole of sialic acid per minute.

Then, to determine the linkage mode of sialic acid, a reaction using PA-lactose as a substrate was performed. The enzyme reaction was performed using the resulting crude enzyme solution and a pyridylaminated sugar chain as the sugar acceptor substrate. As the pyridylaminated sugar chain, pyridylaminated lactose (Galβ1-4Glc-PA, manufactured by Takara Bio Inc.) was used. To 5 µL of the crude enzyme solution, 1.5 µL of 5 mM CMP-NeuAc and 1.5 µL of 10 pmol/µL sugar acceptor substrate were added, followed by reaction at 25°C for 18 hours. After completion of the reaction, the reaction solution was treated at 100°C for 2 minutes to inactivate the enzyme, followed by HPLC to analyze the reaction product. The HPLC was performed with a Shimadzu LC10A (manufactured by Shimadzu Corporation) system and a Takara PALPAK Type R (manufactured by Takara Bio Inc.) analytical column. A reaction solution containing 72 µL of eluent A (100 mM acetate-triethylamine, pH 5.0) was injected into a column equilibrated with 100 mM acetate-triethylamine (pH 5.0) containing 0.15% n-butanol. The pyridylaminated sugar chain was successively eluted using eluent A (100 mM acetate-triethylamine, pH 5.0) and eluent B (100 mM acetate-triethylamine containing 0.5% n-butanol, pH 5.0) with a linear gradient of 30% to 50% eluent B (0 to 20 minutes) and then 100% eluent B (21 to 35 minutes). The analysis was performed under the following conditions: flow rate: 1 mL/min, column temperature: 40°C, detection: fluorescence (Ex: 320 nm, Em: 400 nm). As a result, the JT-SHIZ-119 strain was found to have β-galactoside-α2,6-sialyltransferase activity (Figs. 1-1 to 1-5).

### (2) Bacteriological identification of JT-SHIZ-119 strain by nucleotide sequence analysis of 16S rRNA gene

The genomic DNA extracted from the JT-SHIZ-119 strain in a usual manner was used as a template for PCR to amplify a partial nucleotide sequence of the 16S rRNA gene, thereby determining its nucleotide sequence.

The DNA nucleotide sequence of the 16S rRNA gene in the JT-SHIZ-119 strain was found to have the highest homology, a homology of 99.8%, with the sequence of the 16S rRNA gene of the *Photobacterium leiognathi* type strain ATCC25521. These results identified the JT-SHIZ-119 strain as a microorganism belonging to *Photobacterium leiognathi* belonging to the genus Photobacterium of the family Vibrionaceae.

### Example 2: Cloning and nucleotide sequencing of β-galactoside-α2,6-sialyltransferase derived from JT-SHIZ-119 strain, and expression of the gene in E. coli

### (1) Confirmation of the presence of β-galactoside-α2,6-sialyltransferase gene homologue in JT-SHIZ-119 strain

Genomic Southern hybridization was performed on the JT-SHIZ-119 strain that was found to have β-galactoside-α2,6-sialyltransferase activity, to determine whether there was a homologue for the β-galactoside-α2,6-sialyltransferase gene derived from *Photobacterium damselae* strain JT0160 (Yamamoto, et al., (1996), J. Biochem. 120: 104-110) or for the β-galactoside-α2,6-sialyltransferase gene derived from the JT-ISH-224 strain (PCT/JP2006/315850).

First, to increase the efficiency of hybridization, an attempt was made to use the β-galactoside-α2,6-sialyltransferase gene fragment of the JT-SHIZ-119 strain itself as a probe. Specifically, PCR was performed using nucleotide sequences highly conserved in the β-galactoside-α2,6-sialyltransferase gene derived from *Photobacterium damselae* strain JT0160 or for the β-galactoside-α2,6-sialyltransferase gene derived from the JT-ISH-224 strain as a primer and the genomic DNA of the JT-SHIZ-119 strain as a template to obtain a β-galactoside-α2,6-sialyltransferase gene fragment of the JT-SHIZ-119 strain itself. The primers used for the PCR were as follows:
2,6 consensus 691-701F (5'-GATGATGGTTC-3' (11-mer): SEQ ID NO: 5),
2,6 consensus 1300-1310R (5'-GTCATCATCAA-3' (11-mer): SEQ ID NO: 6),
2,6 consensus 688-702F (5'-TAYGATGATGGTTCW-3' (5-mer): SEQ ID NO: 7), and
2,6 consensus 1288-1311R (5'-YGTCATCATCAANACYTCAAATGA-3' (24-mer): SEQ ID NO: 8).

About 100 µg of genomic DNA was prepared from about 0.5 g of the genomic DNA of the JT-SHIZ-119 strain using a Qiagen Genomic-tip 500/G (manufactured by Qiagen NV) in accordance with the instructions attached to the kit.

The reaction conditions of the PCR were set as follows. In 50 µL of a reaction solution containing 1 µL of genomic DNA of the JT-SHIZ-119 strain as a template, 5 µL of 10 × Ex Taq buffer, 4 µL of each 2.5 mM dNTP, 10 pmol of each primer, and 0.5 µL of Takara Ex Taq (manufactured by Takara Bio Inc.), PCR was carried out as follows: 96°C for 3 min once, 96°C for 1 min, 55°C for 1 min, and 72°C for 2 min 30 cycles, and 72°C for 6 min once, using a Program Temp Control System PC-700 (manufactured by ASTEK Corp.). As a result, the PCR product of approximately 600 bp was amplified by a primer set of a 2,6 consensus 688-702F primer and a 2,6 consensus 1288-1311R primer. The PCR product was cloned into a pCR4TOPO vector (manufactured by Invitrogen Corp.). Ligation was carried out in accordance with instructions attached to the vector kit. The DNA was introduced into *E. coli* TB1 by electroporation, and the plasmid DNA (SHIZ119 688-1311/pCR4) was extracted in a usual manner (Sambrook, et al., 1989, Molecular Cloning, A laboratory manual, 2nd edition). A clone confirmed to have the insert was analyzed using an M13 primer (manufactured by Takara Bio Inc.) to determine the nucleotide sequence of the PCR product from both ends with an ABI PRISM fluorescent sequencer (Model 310 Genetic Analyzer, manufactured by Perkin Elmer, Inc.). As a result, it was confirmed that this DNA fragment has a homology of 95% to the β-galactoside-α2,6-sialyltransferase gene derived from *Photobacterium leiognathi* strain JT-SHIZ-145, a homology of 70% to the β-galactoside-α2,6-sialyltransferase gene derived from *Photobacterium damselae* strain JT0160, and a homology of 70% to the β-galactoside-α2,6-sialyltransferase gene derived from the JT-ISH-224 strain. Using the thus-cloned DNA fragment as a probe, genomic Southern hybridization of the JT-SHIZ-119 strain was performed. Several micrograms of the genomic DNA from the JT-SHIZ-119 strain was digested with a restriction enzyme *Eco*RI, *Hind*III, *Bgl*II, *Kpn*I, *Nde*I, *Pst*I, *Pvu*I, or *Sph*I and fractionated by 0.8% agarose gel electrophoresis. Subsequently, the gel was subjected to alkaline blotting with 0.4 M NaOH to transfer the DNA onto a Hybond-N+ nylon membrane filter (manufactured by GE Health Biosciences). The filter was subjected to Southern hybridization using a homologue fragment (*Eco*RI fragment of SHIZ119 688-1311/pCR4) of the β-galactoside-α2,6-sialyltransferase gene derived from JT-SHIZ-119 strain (GeneBank Accession No. E17028) as a probe. The hybridization experiment was performed using an ECL direct labeling & detection system (manufactured by GE Health Biosciences). The probe was labeled in accordance with instructions attached to the kit. Hybridization was accomplished at 37°C (usually at 42°C) for 4 hours using the hybridization buffer included in the kit, which was supplemented with a 5% (w/v) blocking reagent and 0.5 M NaCl. Washing was performed twice in 0.4% SDS, 0.5 × SSC at 50°C (usually 55°C) for 20 min and once in 2 × SSC at room temperature for 5 min. Signal detection was performed in accordance with instructions attached to the kit. As a result, bands were detected in all restriction enzyme digestion. Among them, in *Sph*I digestion, a band of approximated 3.4 kbp was obtained; and in *Pst*I digestion, a relatively small band of 1.0 kbp was obtained. These results revealed that the JT-SHIZ-119 strain had homologues for the β-galactoside-α2,6-sialyltransferase genes derived from *Photobacterium leiognathi* strain JT-SHIZ-145, *Photobacterium damselae* strain JT0160, and the JT-ISH-224 strain.

### (2) Subcloning of genomic fragment containing β-galactoside-α2,6-sialyltransferase gene homologue from JT-SHIZ-119 strain

As described above, the *Sph*I fragment of 3.4 kbp that appeared to contain the full length of a β-galactoside-α2,6-sialyltransferase gene homologue derived from the JT-SHIZ-119 strain and also appeared easily to be introduced into a plasmid vector was inserted into the plasmid vector pUC18, followed by screening by colony hybridization.

The genomic DNA of the JT-SHIZ-119 strain was digested again with *Sph*I, followed by agarose gel electrophoresis in TAE buffer using a low melting point agarose (SeaPlaqueGTG). A gel piece containing a DNA fragment of around 3.4 kbp was excised, and 200 mM NaCl was added thereto in an equal amount (v/w) with the gel, followed by treatment at 70°C for 10 min to melt the gel. This sample was extracted once with phenol, once with phenol/chloroform, and then once with chloroform, followed by ethanol precipitation to collect a DNA fragment of 1.6 kb. This fragment was ligated to the *Sph*I site of plasmid vector pUC18 that had been dephosphorylated in advance, using a Ligation kit (Takara Bio Inc.). After the ligation reaction, the DNA was transformed into *E. coli* TB1 by electroporation and cultured on an LA agar medium containing 100 µg/mL ampicillin and X-gal (5-bromo-4-chloro-3-indolyl-β-D-galactoside). Three hundred white colonies, into which the DNA fragment appeared to be inserted, were inoculated onto another LA agar medium containing the above-mentioned antibiotic. The surface of each plate on which colonies were formed was put into contact with a Hybond-N+ nylon membrane filter (manufactured by GE Health Biosciences) to transfer the colonies onto the membrane. The colonies were then treated with an alkali in accordance with the instructions attached to the membrane to denature the DNA and fix it on the membrane. This membrane was subjected to colony hybridization using the homologue fragment (*Eco*RI fragment of SHIZ119 688-1311/pCR4) of the β-galactoside-α2,6-sialyltransferase gene derived from the JT-SHIZ-119 strain as a probe. As a result, signals were detected in eight colonies. Note that probe labeling and hybridization conditions were the same as those in the case of using the ECL system as shown above.

These colonies were inoculated into ampicillin-containing LB liquid medium and cultured overnight with shaking at 37°C, followed by plasmid extraction in a usual manner (Sambrook, et al., 1989, Molecular Cloning, A laboratory manual, 2nd edition (hereby incorporated by reference in its entirety)) and restriction enzyme analysis to confirm the insertion of the 3.4 kbp fragment in three clones.

### (3) Determination of the entire nucleotide sequence of β-galactoside-α2,6-sialyltransferase gene homologue derived from JT-SHIZ-119 strain

With respect to three of the plasmids that were confirmed above to carry the insert DNA, nucleotide sequences at both ends of the 3.4 kbp *Sph*I fragment were determined using M13 primers (Takara Bio Inc.) with an ABI PRISM fluorescent sequencer (Model 310 Genetic Analyzer, manufactured by Perkin Elmer, Inc.). The resulting DNA sequences were translated into amino acid sequences using genetic information processing software GENETYX Ver. 7 (available from Genetyx Corporation), and identity search of the amino acid sequences was performed with the BLAST program against the GeneBank database of the National Center for Biotechnology Information (NCBI). The results elucidated that the amino acid sequence translated from one of the DNA sequences showed significant homology with the amino acid sequence of β-galactoside-α2,6-sialyltransferase derived from *Photobacterium damselae* strain JT0160. The orientation of the region showing the homology suggested that the 3.4 kbp *Sph*I fragment contained the entire β-galactoside-α2,6-sialyltransferase gene homologue derived from the JT-SHIZ-119 strain.

Next, to determine completely the DNA sequence of this enzyme gene homologue derived from the JT-SHIZ-119 strain, the following five primers:
SHIZ-119-26 412-431F (5'-GAGTATTCACAGAATGAGCG-3' (20-mer): SEQ ID NO: 9),
SHIZ-119-26 521-540F (5'-CACAAGAACTTGTAGATGCA-3' (20-mer): SEQ ID NO: 10),
SHIZ-119-26 325-344F (5'-GTTGTTGCCCCAACACTAGA-3' (20-mer): SEQ ID NO: 11),
SHIZ-119-26 640-659F (5'-CTAGGTAGAGAGCATGATCT-3' (20-mer): SEQ ID NO: 12), and
SHIZ-119-26 671-690F (5'-GTCATCCAAGAGGAGGAATT-3' (20-mer): SEQ ID NO: 13)
were synthesized based on the DNA sequence obtained from the 3.4 kbp *Sph*I fragment and were used for nucleotide sequencing.

Using these primers, nucleotide sequencing was performed to obtain the sequence of SEQ ID NO: 3 in the Sequence Listing. This sequence is the entire nucleotide sequence of the open reading frame (ORF) of the β-galactoside-α2,6-sialyltransferase gene homologue derived from the JT-SHIZ-119 strain. The ORF of the β-galactoside-α2,6-sialyltransferase gene homologue derived from *Photobacterium sp.* strain JT-SHIZ-119 was composed of 1536 base pairs and encoded 511 amino acids. This amino acid sequence is shown in SEQ ID NO: 2 in the Sequence Listing. The analysis of DNA and amino acid sequences using GENETYX Ver. 7 showed that the DNA sequence of the β-galactoside-α2,6-sialyltransferase gene homologue derived from the JT-SHIZ-119 strain had a homology of 95% with the β-galactoside-α2,6-sialyltransferase gene derived from *Photobacterium leiognathi* strain JT-SHIZ-145 in both the nucleotide and amino acid sequences; a homology of 67% in nucleotide sequence and a homology of 66% in amino acid sequence with the β-galactoside-α2,6-sialyltransferase gene derived from *Photobacterium damselae* strain JT0160; and a homology of 64% in nucleotide sequence and a homology of 56% in amino acid sequence with the β-galactoside-α2,6-sialyltransferase gene derived from the JT-ISH-224 strain.

### (4) Construction of expression vector for β-galactoside-α2,6-sialyltransferase gene homologue derived from JT-SHIZ-119 strain

To investigate whether the cloned gene encodes a protein having sialyltransferase activity, the full length of the gene homologue and a gene lacking the region encoding the N-terminal signal peptide were each integrated into an expression vector to produce a protein in *E. coli* cells, and the activities of the expressed proteins were measured.

The amino acid sequence encoding the β-galactoside-α2,6-sialyltransferase gene homologue derived from the JT-SHIZ-119 strain was analyzed with genetic information processing software GENETYX Ver. 7 to estimate that 15 amino acids at the N-terminus would constitute the signal peptide. Accordingly, a primer pair for cloning the full-length gene (in this example, referred to as SHIZ119-N0C0):
SHIZ119 N0 Bsp (5'-GCGCGTCATGAAAAGAATATTTTGTTTAGTCTCTGC-3' (36-mer): SEQ ID NO: 14) and
SHIZ119 C0 Bam (5'-ATTAAGGATCCCTAATATTGAGCAATACAC-3' (30-mer): SEQ ID NO: 15), and
a primer pair for cloning a gene encoding a protein lacking the amino acids of the signal peptide region (in this example, referred to as SHIZ119-N1C0):
SHIZ119 N1 Pci (5'-GGGACATGTGTAATGATAATCAGAATACAG-3' (30-mer): SEQ ID NO: 16) and
SHIZ119 C0 Bam (5'-ATTAAGGATCCCTAATATTGAGCAATACAC-3' (30-mer): SEQ ID NO: 15)
were designed and synthesized. PCR was performed using these primers and using the plasmid containing the 3.4 kbp SphI fragment as a template to amplify the β-galactoside-α2,6-sialyltransferase gene homologue derived from the JT-SHIZ-119 strain to be integrated into an expression vector. The reaction conditions for the PCR were set as follows. In 50 µL of a reaction solution containing 500 ng of template DNA, 5 µL of 10 × PyroBest buffer II, 4 µL of each 2.5 mM dNTP, 50 pmol of each primer, and 0. 5 µL of PyroBest DNA Polymerase (manufactured by Takara Bio Inc.), PCR was carried out as follows: 96°C for 3 min once, 96°C for 1 min, 55°C for 1 min, and 72°C for 2 min 10 cycles, and 72°C for 6 min once, using a Program Temp Control System PC-700 (manufactured by ASTEK Corp.). As a result, PCR products of approximately 1.5 kb and 1.45 kb were amplified for SHIZ119-N0C0 and SHIZ119-N1C0, respectively. These PCR products were each cloned into vector pCR4BluntTOPO (manufactured by Invitrogen Corp.). Ligation was carried out in accordance with instructions attached to the vector kit. Each DNA was introduced into *E. coli* TB1 by electroporation, and the plasmid DNA was extracted in a usual manner (Sambrook, et al., 1989, Molecular Cloning, A laboratory manual, 2nd edition). Clones confirmed to have the insert were each analyzed by PCR with M13 primers (manufactured by Takara Bio Inc.) to determine the nucleotide sequence of each PCR product from both ends using an ABI PRISM fluorescent sequencer (Model 310 Genetic Analyzer, manufactured by Perkin Elmer, Inc.). The results showed that mutation-free SHIZ119-N0C0 and SHIZ119-N1C0 were cloned.

Clones of SHIZ119-N0C0 and SHIZ119-N1C0 whose nucleotide sequences were confirmed were double-digested with restriction enzymes BspHI and BamHI (for SHIZ119-N0C0) or *Pci*I and *Bam*HI (for SHIZ119-N1C0), followed by gel purification of each DNA fragment as described above. As the *E. coli* expression vector, pTrc99A (manufactured by Pharmacia LKB) was used. This vector was double-digested with restriction enzymes *Nco*I and *Bam*HI, followed by gel purification. This vector was ligated with the DNA fragment of SHIZ119-N0C0 or SHIZ119-N1C0 prepared as described above using a Ligation Kit (manufactured by Takara Bio Inc.) and transformed into *E. coli* TB1. In a usual manner, the plasmid DNA was extracted and subjected to restriction enzyme analysis to confirm the integration of the DNA fragment into the expression vector, thereby completing SHIZ 119-N0C0/pTrc99A or SHIZ119-N1C0/pTrc99A.

### (5) Expression induction and activity measurement

An induction experiment of protein expression was performed using two types of expression vectors prepared above. A single colony of *E. coli* TB1 having the expression vector pTrc99A containing each clone was inoculated into LB medium (6 mL) containing an antibiotic, ampicillin (final concentration 100 µg/mL), and pre-cultured at 30°C until the absorbance at 600 nm reached about 0.5, followed by addition of IPTG (isopropyl-β-D(-)-thiogalactopyranoside), manufactured by Wako Pure Chemical Industries, Ltd.) at a final concentration of 1 mM to initiate expression induction. After culturing overnight with shaking at 30°C, the cells in 2 mL of the culture solution were collected by centrifugation. These cells were suspended in 400 µL of 20 mM Bis-Tris buffer (pH 6.0) containing 0.336% Triton X-100 and sonicated under ice cooling. The resulting homogenate was subjected to measurement of sialyltransferase activity as a crude enzyme solution. The measurement was performed as described in J. Biochem., 120, 104-110 (1996) (hereby incorporated by reference in its entirety). Specifically, CMP-NeuAc (70 nmol, containing about 20000 cpm CMP-NeuAc in which NeuAc was labeled with ¹⁴C; NeuAc represents *N*-acetylneuraminic acid) as a sugar donor substrate, 0.5 M NaCl, 120 mM lactose as a sugar acceptor substrate, and 5 µL of the crude enzyme solution prepared as described above were mixed, followed by reaction at 30°C for 30 minutes. Subsequently, 1.97 mL of 5 mM phosphate buffer (pH 6.8) was added to quench the reaction, which was then applied to a Dowex 1 × 8 (PO₄³⁻ form, 0.2 × 2 cm, manufactured by Bio-Rad Laboratories, Inc.) column. The radioactivity contained in the reaction product contained in the eluate from this column, that is, sialyllactose, was measured to calculate the enzyme activity. The measurement was performed in duplicate. The results showed that the crude enzyme solution from *E. coli* containing SHIZ119-N0C0 or SHIZ119-N1C0 had the ability to transfer ¹⁴C-labeled NeuAc in the sugar donor CMP-NeuAc to the sugar acceptor substrate lactose, i.e., sialyltransferase activity. A homogenate prepared from *E. coli* transformed with a pTrc99A vector not containing insert was used as a negative control. The radioactivity of the control was 170 cpm, whereas those in the cases of SHIZ119-N0C0 and SHIZ119-N1C0 were 8560 to 8990 cpm and 7786 to 8446 cpm, respectively.

The results described above revealed that the cloned homologues were genes encoding sialyltransferase.

**[Table 1]**

| Enzyme activity of crude enzyme extraction solution prepared from *E.* coli transformed with SHIZ119-N1C0/pTrc99A | | |
|---|---|---|
| | Transferred NeuAc (cpm) | |
| Without acceptor | 170 | |
| With acceptor | 7786 | 8446 |

### (6) Confirmation of β-galactoside-α2,6-sialyltransferase activity

It was investigated whether sialyltransferase expressed by *E. coli* transformed with SHIZ119-N1C0/pTrc99A in Example 2(5) above had β-galactoside-α2,6-sialyltransferase activity. As in Example 1, the enzyme reaction was performed using pyridylaminated lactose (Galβ1-4Glc-PA, PA-Sugar Chain 026, manufactured by Takara Bio Inc.) as a sugar acceptor. As a result, PA-6'-sialyllactose (Neu5Aca2-6Galβ1-4Glc-PA) was detected, as in Example 1. These results demonstrated that the β-galactoside-α2,6-sialyltransferase gene from *Photobacterium* sp. strain JT-SHIZ-119 was cloned and expressed in *E. coli.*

### Example 3: Extraction and purification of β-galactoside-α2,6-sialyltransferase from E. coli TB1 having expression vector pTrc99A containing SHIZ119-N1C0 clone

### (1) Extraction and purification

Cells were collected with a loop from colonies of *E. coli* TB1 having the expression vector pTrc99A containing the SHIZ119-N1C0 clone, which had been subcultured on an LBAmp plate medium, and were inoculated into 10 mL of 6 mL-LB liquid medium supplemented with 30 µL of × 200 ampicillin (400 mg/20 mL) and cultured with shaking at 30°C at 180 rpm for 8 hours.

Main culturing was performed by the following procedure: 300 mL of LB medium supplemented with 1.5 mL of × 200 ampicillin (400 mg/20 mL) and 300 µL of 1 M IPTG (1.192 g/5 mL) was charged in a 1000-mL baffle flask. The same medium was prepared in 9 flasks (2.7 L in total). Each flask was inoculated with 12 mL of the preculture solution obtained above, followed by culturing with shaking at 30°C at 180 rpm for 24 hours. The culture solution was centrifuged to collect the cells.

The cells were suspended in 990 mL of 20 mM Bis-Tris buffer (pH 7.0) containing 0.3% Triton X-100 to give a concentration of 1.6 g/26 mL and were sonicated under ice cooling. The cell homogenate was centrifuged at 4°C at 100,000 × g for 1 hour to obtain the supernatant.

This crude enzyme solution was applied to a HiLoad 26/10 Q Sepharose HP (manufactured by Amersham) anion exchange column equilibrated with 20 mM Bis-Tris buffer (pH 6.0) containing 0.3% Triton X-100, and was eluted by a linear gradient from 20 mM Bis-Tris buffer (pH 7.0) containing 0.3% Triton X-100 to a buffer containing 1 M sodium chloride to collect an enzymatically active fraction eluted at around 0.36 M sodium chloride concentration.

The collected fraction was diluted with 20 mM phosphate buffer (pH 6.0) and applied to hydroxyapatite (manufactured by Bio-Rad Laboratories, Inc.) equilibrated in advance with 20 mM phosphate buffer (pH 6.0) containing 0.3% Triton X-100, followed by elution with a linear gradient from 20 mM phosphate buffer (pH 6.0) containing 0.3% Triton X-100 to 500 mM phosphate buffer (pH 6.0) containing 0.336% Triton X-100 thereby to collect an enzymatically active fraction eluted at around 125 mM phosphate buffer concentration.

Subsequently, this enzymatically active fraction was applied to an MonoQ 5/50 GL (manufactured by Amersham) anion exchange column and was eluted with a linear gradient from 20 mM Bis-Tris buffer (pH 6.0) containing 0.336% Triton X-100 to the buffer containing 1 M sodium chloride thereby to collect an enzymatically active fraction.

The enzymatically active fraction was electrophoresed on an SDS-polyacrylamide gel (the concentration of the acrylamide gel: 12.5%). The target enzyme was detected as a single band with a molecular weight of about 53,000.

Table 2 shows the enzyme activity of the sample after each of the purification steps mentioned above as to purification of β-galactoside-α2,6-sialyltransferase of the SHIZ 119-N1C0 clone from the crude enzyme solution. The enzyme activity was measured by the method described in J. Biochem., 120, 104-110 (1996), as in Example 1. The amount of the protein was measured using a Coomassie Protein Assay Reagent (manufactured by Pierce) in accordance with the instruction manual attached thereto. One enzyme unit (1 U) was defined as the amount of enzyme required to transfer one micromole of sialic acid per minute.

**[Table 2]**

| Purification of recombinant β-galactoside-α2,6-sialyltransferase N1C0 from *E. coli* transformed with SHIZ119-N1 C0/pTrc99A | | | | | | |
|---|---|---|---|---|---|---|
| Sample | Volume | Total protein | Total activity | Specific activity | Yield | Degree of purification |
| | (mL) | (mg) | (U) | (U/mg) | (%) | (fold) |
| Crude enzyme solution | 370 | 850 | 4646.4 | 5.5 | 100 | 1 |
| Q-sepharose | 30 | 84.3 | 3001.6 | 35.6 | 64.6 | 6.5 |
| Mono-Q | 13 | 63.8 | 2308.3 | 36.2 | 49.7 | 6.6 |
| HAP | 16 | 29.8 | 2471.3 | 82.9 | 53.2 | 15.2 |

### Example 4: Optimum pH and optimum temperature for enzyme activity of recombinant β-galactoside-α2,6-sialyltransferase N1C0 derived from JT-SHIZ-119 strain

The optimum pH and the optimum temperature of the recombinant β-galactoside-α2,6-sialyltransferase SHIZ119-N1C0 derived from the JT-SHIZ-119 strain were investigated using the purified enzyme prepared in Example 3.

### (1) Optimum pH for enzyme activity of JT-SHIZ-119-derived recombinant β-galactoside-α2,6-sialyltransferase N1C0

Acetate buffer (pH 4.0 to 5.0), cacodylate buffer (pH 5.0 to 6.0), Bis-Tris buffer (pH 6.0 to 7.0), phosphate buffer (pH 7.0 to 8.0), TAPS buffer (pH 8.0 to 9.0), CHES buffer (pH 9.0 to 10.0), and CAPS buffer (pH 10.0 to 11.0) were each prepared and were used for enzyme activity measurement at 30°C at various pH levels.

As shown in Fig. 2-1, the enzyme activity is the highest at a pH of 5.0. Note that the enzyme activity at each pH is shown as a relative activity to an enzyme activity represented by 100 at a pH of 5.0.

### (2) Optimum temperature for enzyme activity of JT-SHIZ-119-derived recombinant β-galactoside-α2,6-sialyltransferase N1C0

The enzyme activity was measured at every increment of 5°C starting from 5°C up to 50°C using cacodylate buffer (pH 5.0).

As shown in Fig. 2-2, the enzyme activity is the highest at 35°C. Note that the enzyme activity at each temperature is shown as a relative activity to an enzyme activity represented by 100 at 35°C.

### Example 5: Sugar acceptor substrate specificity of recombinant β-galactoside-α2,6-sialyltransferase N1C0 derived from JT-SHIZ-119 strain

Sialic acid transfer reaction was performed using the purified enzyme, JT-SHIZ-119-derived recombinant β-galactoside-α2,6-sialyltransferase SHIZ119-N1C0, prepared in Example 3 and using various monosaccharides/disaccharides as sugar acceptor substrates. The reaction was performed by the method described in J. Biochem., 120, 104-110 (1996).

The monosaccharides used as sugar acceptor substrates were the following eight types: methyl-α-D-galactopyranoside (Gal-α-OMe), methyl-β-D-galactopyranoside (Gal-β-OMe), methyl-α-D-glucopyranoside (Glc-α-OMe), methyl-β-D-glucopyranoside (Glc-β-OMe), methyl-α-D-mannopyranoside (Man-α-OMe), methyl-β-D-mannopyranoside (Man-β-OMe), *N*-acetylgalactosamine (GalNAc), and *N*-acetylglucosamine (GlcNAc). The disaccharides used were the following three types: lactose (Gal-β1,4-Glc), *N-*acetyllactosamine (Gal-β1,4-GlcNAc), and Gal-β1,3-GalNAc.

It was revealed that sialic acid was efficiently transferred to methyl-β-D-galactopyranoside, *N*-acetylgalactosamine, lactose, *N*-acetyllactosamine, and Gal-β1,3-GalNAc among the 11 types of monosaccharides and disaccharides used as sugar acceptor substrates in this experiment (Table 3). Note that the relative activity for each acceptor substrate is based on the sialyltransferase activity represented by 100 for lactose.

**[Table 3]**

| Transfer of sialic acid to monosaccharides and disaccharides by recombinant β-galactoside-α2,6-sialyltransferase N1C0 purified from *E. coli* transformed with SHIZ119-N1 C0/pTrc99A | | |
|---|---|---|
| Sugar acceptor substrate | Transferred NeuAc (nmol/min) | Relative activity (%) |
| Methyl-α-D-galactopyranoside | 0.04 | 1 |
| Methyl-β-D-galactopyranoside | 1.1 | 38 |
| Methyl-α-D-glucopyranoside | <0.01 | - |
| Methyl-β-D-glucopyranoside | <0.01 | - |
| Methyl-α-D-mannopyranoside | <0.01 | - |
| Methyl-β-D-mannopyranoside | 0.04 | 1 |
| *N*-Acetylgalactosamine | 0.29 | 10 |
| *N*-Acetylglucosamine | <0.01 | - |
| Lactose (Gal-β-1,4-Glc) | 2.88 | 100 |
| *N*-Acetyllactosamine (Gal-β-1,4-GlcNAc) | 2.96 | 103 |
| Gal-β-1,3-GalNAc | 1.68 | 58 |

### Example 6: Confirmation and substrate specificity of neuraminidase activity of JT-SHIZ-119-derived recombinant β-galactoside-α2,6-sialyltransferase N1C0

The neuraminidase activity was measured using JT-SHIZ-119-derived recombinant β-galactoside-α2,6-sialyltransferase.

### (1) Confirmation of neuraminidase activity of JT-SHIZ-119-derived recombinant β-galactoside-α2,6-sialyltransferase N1C0

In the process described in Example 2(6), the reaction using the purified enzyme solution of the JT-SHIZ-119- derived recombinant β-galactoside-α2,6-sialyltransferase prepared in Example 3 was performed for a long time. The signal of the reaction product (PA-6'-sialyllactose, retention time: 4.02 min) decreased and the signal of the PA-lactose (retention time: 3.73 min), which was the original sugar acceptor substrate, increased in accordance with an increase in reaction time (Fig. 3-1).

These results reveals that JT-SHIZ-119-derived recombinant β-galactoside-α2,6-sialyltransferase SHIZ119-N1C0 also has neuraminidase activity.

### (2) Substrate specificity of neuraminidase activity of JT-SHIZ-119-derived recombinant β-galactoside-α2,6-sialyltransferase N1C0

To reveal the specificity of the neuraminidase activity of the JT-SHIZ-119-derived recombinant β-galactoside-α2,6-sialyltransferase N1C0, reactions using a PA-sugar chain shown in Table 4, to which sialic acid is linked via α2,3-, α2,6-, or α2,8-linkage (PA-Sugar Chain 029, PA-Sugar Chain 023, or PA-Sugar Chain 034, manufactured by Takara Bio Inc.) as a substrate were performed.

To a purified enzyme solution of the JT-SHIZ-119-derived recombinant β-galactoside-α2,6-sialyltransferase N1C0 (equivalent to 0.6 U as sialyltransferase activity), 1.5 µL of a 10 pmol/µL PA-sugar chain was added, followed by reaction at 30°C for 18 hours. After completion of the reaction, the reaction solution was treated at 100°C for 2 minutes to inactivate the enzyme, followed by HPLC to analyze the reaction product. In the case of using PA-Sugar Chain 023 as the substrate, the pyridylaminated sugar chain was eluted and analyzed under the same conditions as those in Example 1. In the case of using PA-Sugar Chain 029 or PA-Sugar Chain 034 as the substrate, the elution was performed using eluent A (100 mM acetate-triethylamine, pH 5.0) and eluent B (100 mM acetate-triethylamine containing 0.5% n-butanol, pH 5.0) with a linear gradient of 0 to 100% eluent B (0 to 35 min), 100% eluent B (35 to 50 min), and then a linear gradient of 100% to 30% eluent B (51 to 75 min). The analysis was performed under the conditions of Example 1. Figs. 3-2 to 3-8 show the results. The neuraminidase activity of the JT-SHIZ-119-derived recombinant β-galactoside-α2,6-sialyltransferase SHIZ119-N1C0 is specific to sialic acid of α2,6-linkage.

**[Table 4]**

| PA-sugar chain used in analysis of specificity of sialidase activity | |
|---|---|
| Type of PA-sugar chain | Name |
| | Structure |
| PA-Sugar Chain 001 | *N*-Acetyllactosamine type, biantennary |
| | Gal β 1-4GlcNAc β 1-2Man α 1 6 Man β 1-4GlcNAc β 1-4GlcNAc-PA 3 Gal β 1-4GlcNAc β 1-2Man α 1 |
| PA-Sugar Chain 021 | *N*-Acetyllactosamine type, monosialylated biantennary |
| | Neu5Acα2-6Galβ1-4GlcNAcβ1-2Manα1 6 Manβ1-4GlcNAcβ1-4GlcNAc-PA 3 Galβ1-4GlcNAcβ1-2Manα1 |
| PA-Sugar Chain 022 | *N*-Acetyllactosamine type, monosialylated biantennary |
| | Gal β 1-4GlcNAc β 1-2Man α 1 6 Man β 1-4GlcNAc β 1-4GlcNAc-PA 3 Neu5Ac α 2-6Gal β 1-4GlcNAc β 1-2Man α 1 |
| PA-Sugar Chain 023 | *N*-Acetyllactosamine type, disialylated biantennary |
| | Neu5Ac α 2-6Gal β 1-4GlcNAc β 1-2Man α 1 6 Man β 1-4GlcNAc β 1-4GlcNAc-PA 3 Neu5Ac α 2-6Gal β 1-4GlcNAc β 1-2Man α 1 |
| PA-Sugar Chain 026 | Lactose |
| | Gal *β* 1-4Glc-PA |
| PA-Sugar Chain 028 | asialo GM1-tetrasaccharide |
| | Gal *β* 1-3GalNac *β* 1-4Gal *β* 1-4Glc-PA |
| PA-Sugar Chain 029 | GM3-NeuSAc-trisaccharide |
| | Neu5Ac *α* 2-3Gla *β* 1-4Glc-PA |
| PA-Sugar Chain 032 | GM1-pentasaccharide |
| | Gal β 1-3GalNAc β 1-4Gal β 1-4Glc-PA 3 Neu5Ac α 2 |
| PA-Sugar Chain 034 | GD1b-hexasaccharide |
| | 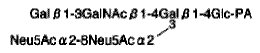 Gal β 1-3GalNAc β 1-4Gal β 1-4Glc-PA 3 Neu5Ac α 2-8Neu5Ac α 2 |

### Example 7: Optimum pH and optimum temperature for neuraminidase activity of recombinant β-galactoside-α2,6-sialyltransferase N1C0 derived from JT-SHIZ-119 strain

The optimum pH and the optimum temperature for neuraminidase activity of the recombinant β-galactoside-α2,6-sialyltransferase SHIZ119-N1C0 derived from the JT-SHIZ-119 strain were investigated using the purified enzyme prepared in Example 3.

### (1) Optimum pH for neuraminidase activity of JT-SHIZ-119-derived recombinant β-galactoside-α2,6-sialyltransferase N1C0

The enzyme activity at various pH levels was measured at 35°C with the buffer used in Example 4.

The results are shown in Fig. 4-1. The neuraminidase activity is the highest at a pH of 6.0. Note that the enzyme activity at each pH is shown as a relative activity to an enzyme activity represented by 100 a pH of 6.0.

### (2) Optimum temperature for neuraminidase activity of JT-SHIZ-119-derived recombinant β-galactoside-α2,6-sialyltransferase N1C0

The neuraminidase activity was measured at every increment of 5°C starting from 5°C up to 50°C using cacodylate buffer (pH 6.0).

As shown in Fig. 4-2, the enzyme activity is the highest at 35°C. Note that the enzyme activity at each temperature is shown as a relative activity to an enzyme activity represented by 100 at 35°C.

### INDUSTRIAL APPLICABILITY

The present invention provides a novel β-galactoside-α2,6-sialyltransferase and a nucleic acid encoding the same, which provides a means for synthesizing and producing sugar chains which have been shown to have important functions *in vivo.* In particular, sialic acid is often located at the nonreducing termini of sugar chains of complex carbohydrates *in vivo* and is a very important sugar from the viewpoint of sugar chain functions. Accordingly, sialyltransferase is one of the most demanded enzymes among glycosyltransferases. The novel sialyltransferase of the present invention can be used for the development of pharmaceuticals, functional foods and other products to which sugar chains are applied.

The polypeptide encoded by the above-described nucleic acid also has neuraminidase activity, which specifically cleaves sialic acid of α2,6-linkage and can be used for quantitative measurement of sialic acid of α 2,6-linkage contained *in vivo.*

### SEQUENCE LISTING FREE TEXT

SEQ ID NO: 1: nucleic acid sequence encoding SHIZ119-N0C0
SEQ ID NO: 2: amino acid sequence of SHIZ119-N0C0
SEQ ID NO: 3: nucleic acid sequence encoding SHIZ119-N1C0
SEQ ID NO: 4: amino acid sequence of SHIZ119-N1C0
SEQ ID NO: 5: primer 2,6 consensus 691-701F
SEQ ID NO: 6: primer 2,6 consensus 1300-1310R
SEQ ID NO: 7: primer 2,6 consensus 688-702F
SEQ ID NO: 8: primer 2,6 consensus 1288-1311R
SEQ ID NO: 9: primer SHIZ-119-26 412-431F
SEQ ID NO: 10: primer SHIZ-119-26 521-540F
SEQ ID NO: 11: primer SHIZ-119-26 325-344F
SEQ ID NO: 12: primer SHIZ-119-26 640-659F
SEQ ID NO: 13: primer SHIZ-119-26 671-690F
SEQ ID NO: 14: primer SHIZ119 N0 Bsp
SEQ ID NO: 15: primer SHIZ119 C0 Bam
SEQ ID NO: 16: primer SHIZ11 N1 Pci
SEQ ID NO: 17: FLAG^{™} tag

## Claims

1. An isolated protein comprising an amino acid sequence selected from the group consisting of SEQ ID NO: 2, SEQ ID NO: 4, and amino acids 16 to 511 of SEQ ID NO: 2.

2. An isolated protein having neuraminidase activity and/or β-galactoside-α2,6-sialyltransferase activity, wherein the protein comprises:
(a) an amino acid sequence comprising deletion, substitution, insertion, and/or addition of one or more amino acids in an amino acid sequence selected from the group consisting of SEQ ID NO: 2, SEQ ID NO: 4, and amino acids 16 to 511 of SEQ ID NO: 2; or
(b) an amino acid sequence having an amino acid identity of 97% or more with an amino acid sequence selected from the group consisting of SEQ ID NO: 2, SEQ ID NO: 4, and amino acids 16 to 511 of SEQ ID NO: 2.

3. An isolated protein encoded by a nucleic acid comprising a nucleotide sequence selected from the group consisting of SEQ ID NO: 1, SEQ ID NO: 3, and nucleotides 46-1536 of SEQ ID NO: 1.

4. An isolated protein having neuraminidase activity and/or β-galactoside-α2,6-sialyltransferase activity, wherein the protein is encoded by a nucleic acid comprising:
(a) a nucleotide sequence comprising deletion, substitution, insertion, and/or addition of one or more nucleotides in a nucleotide sequence selected from the group consisting of SEQ ID NO: 1, SEQ ID NO: 3, and nucleotides 46-1536 of SEQ ID NO: 1;
(b) a nucleotide sequence having an identity of 97% or more with a nucleotide sequence selected from the group consisting of SEQ ID NO: 1, SEQ ID NO: 3, and nucleotides 46-1536 of SEQ ID NO: 1; or,
(c) a nucleotide sequence hybridizable under stringent conditions with the complementary strand of a nucleotide sequence selected from the group consisting of SEQ ID NO: 1, SEQ ID NO: 3, and nucleotides 46-1536 of SEQ ID NO: 1.

5. The isolated protein according to any one of Claims 1 to 4, wherein the protein has neuraminidase activity wherein said neuraminidase activity is an activity that selectively cleaves a sialic acid residue located at the nonreducing terminus of a sugar chain with α2,6-linkage.

6. The isolated protein according to any one of Claims 1 to 4, wherein the protein has an optimum reaction pH of 5.0 to 7.0 for the neuraminidase activity.

7. The isolated protein according to any one of Claims 1 to 4, wherein the protein has an optimum reaction temperature of 25°C to 40°C for the neuraminidase activity.

8. The isolated protein according to any one of Claims 1 to 4, wherein the protein has an optimum reaction pH of 4.0 to 9.0 for the β-galactoside-α2,6-sialyltransferase activity.

9. The isolated protein according to any one of Claims 1 to 4, wherein the protein has an optimum reaction temperature of 30°C to 40°C for the β-galactoside-α2,6-sialyltransferase activity.

10. The isolated protein according to any one of Claims 1 to 4, wherein the protein is derived from a microorganism belonging to the genus Photobacterium.

11. An isolated nucleic acid encoding a protein comprising an amino acid sequence selected from the group consisting of SEQ ID NO: 2, SEQ ID NO: 4, and amino acids 16 to 511 of SEQ ID NO: 2.

12. An isolated nucleic acid encoding a protein having neuraminidase activity and/or β-galactoside-α,2,6-sialyltransferase activity, wherein the nucleic acid encodes the protein comprising:
(a) an amino acid sequence comprising deletion, substitution, insertion, and/or addition of one or more amino acids in an amino acid sequence selected from the group consisting of SEQ ID NO: 2, SEQ ID NO: 4, and amino acids 16 to 511 of SEQ ID NO: 2; or
(b) an amino acid sequence having an amino acid identity of 97% or more with an amino acid sequence selected from the group consisting of SEQ ID NO: 2, SEQ ID NO: 4, and amino acids 16 to 511 of SEQ ID NO: 2.

13. The isolated nucleic acid comprising a nucleotide sequence selected from the group consisting of SEQ ID NO: 1, SEQ ID NO: 3, and nucleotides 46-1536 of SEQ ID NO: 1.

14. An isolated nucleic acid encoding a protein having neuraminidase activity and/or β-galactoside-α2,6-sialyltransferase activity, wherein the nucleic acid comprises:
(a) a nucleotide sequence comprising deletion, substitution, insertion, and/or addition of one or more nucleotides in a nucleotide sequence selected from the group consisting of SEQ ID NO: 1, SEQ ID NO: 3, and nucleotides 46-1536 of SEQ ID NO: 1;
(b) a nucleotide sequence having an identity of 97% or more with a nucleotide sequence selected from the group consisting of SEQ ID NO: 1, SEQ ID NO: 3, and nucleotides 46-1536 of SEQ ID NO: 1; or,
(c) a nucleotide sequence hybridizable under stringent conditions with the complementary strand of a nucleotide sequence selected from the group consisting of SEQ ID NO: 1, SEQ ID NO: 3, and nucleotides 46-1536 of SEQ ID NO: 1.

15. An expression vector comprising the nucleic acid according to any one of Claims 11 to 14.

16. A host cell transformed with the expression vector according to Claim 15.

17. A method of producing a recombinant protein having neuraminidase activity and/or β-galactoside-α2,6-sialyltransferase activity, wherein the method comprises the steps of:
1) transforming a host cell with an expression vector including the nucleic acid according to any one of Claims 11 to 14;
2) culturing the resulting transformed cell; and
3) isolating a protein having neuraminidase activity and/or β-galactoside-α2,6-sialyltransferase activity from the cultured transformed cell or the culture supernatant thereof.

18. An antibody specifically recognizing the protein according to any one of Claims 1 to 10.


SEQUENCE LISTING

<110>  Japan Tobacco Inc.

<120>  A novel protein and a gene encoding thereof

<130>  FA0392-10051PCT

<150>  JP 2009-141312
<151>  2009-06-12

<160>  17

<170>  PatentIn version 3.1

<210>  1
<211>  1536
<212>  DNA
<213>  Photobacterium leiognathi

<220>
<221>  CDS
<222>  (1)..(1536)
<223>

<400>  1
atg aaa aga ata ttt tgt tta gtc tct gct att tta tta tca gca tgt      48
Met Lys Arg Ile Phe Cys Leu Val Ser Ala Ile Leu Leu Ser Ala Cys
1               5                   10                  15

aat gat aat cag aat aca gta gat gta gtt gta tct act gtg aat gat      96
Asn Asp Asn Gln Asn Thr Val Asp Val Val Val Ser Thr Val Asn Asp
            20                  25                  30

aac gtt att gaa aat aat act tac caa gtt aaa ccc att gat act cca      144
Asn Val Ile Glu Asn Asn Thr Tyr Gln Val Lys Pro Ile Asp Thr Pro
        35                  40                  45

act act ttt gat tcc tat tct tgg ata caa aca tgc ggt act cca ata      192
Thr Thr Phe Asp Ser Tyr Ser Trp Ile Gln Thr Cys Gly Thr Pro Ile
    50                  55                  60

tta aaa gac gat gag aag tac tct ttg agt ttt gac ttt gtt gca cct      240
Leu Lys Asp Asp Glu Lys Tyr Ser Leu Ser Phe Asp Phe Val Ala Pro
65                  70                  75                  80

gag tta gat caa gat gaa aaa ttc tgc ttt gag ttt act ggt gat gtt      288
Glu Leu Asp Gln Asp Glu Lys Phe Cys Phe Glu Phe Thr Gly Asp Val
                85                  90                  95

gat ggt aag cgt tat gtt acc caa act aat ttg act gtt gtt gcc cca      336
Asp Gly Lys Arg Tyr Val Thr Gln Thr Asn Leu Thr Val Val Ala Pro
            100                 105                 110

aca cta gaa gta tat gtg gat cat gca tca ttg cca tca tta cag cag      384
Thr Leu Glu Val Tyr Val Asp His Ala Ser Leu Pro Ser Leu Gln Gln
        115                 120                 125

tta atg aaa ata atc caa cag aaa aat gag tat tca cag aat gag cgc      432
Leu Met Lys Ile Ile Gln Gln Lys Asn Glu Tyr Ser Gln Asn Glu Arg
    130                 135                 140

ttt att tct tgg gga cga att gga ctt aca gaa gat aac gca gaa aaa      480
Phe Ile Ser Trp Gly Arg Ile Gly Leu Thr Glu Asp Asn Ala Glu Lys
145                 150                 155                 160

tta aat gcc cat ata tat cca tta gct gga aat aac aca tca caa gaa      528
Leu Asn Ala His Ile Tyr Pro Leu Ala Gly Asn Asn Thr Ser Gln Glu
                165                 170                 175

ctt gta gat gca gtt att gac tat gct gac tct aaa aat cga tta aat      576
Leu Val Asp Ala Val Ile Asp Tyr Ala Asp Ser Lys Asn Arg Leu Asn
            180                 185                 190

cta gag ctt aat acg aat aca gcg cat tct ttt cca aat cta gca cca      624
Leu Glu Leu Asn Thr Asn Thr Ala His Ser Phe Pro Asn Leu Ala Pro
        195                 200                 205

ata tta cgt ata ata tca tca aag agt aat ata cta att tca aat att      672
Ile Leu Arg Ile Ile Ser Ser Lys Ser Asn Ile Leu Ile Ser Asn Ile
    210                 215                 220

aat tta tat gat gat ggt tct gca gag tat gtt aac ctt tat aac tgg      720
Asn Leu Tyr Asp Asp Gly Ser Ala Glu Tyr Val Asn Leu Tyr Asn Trp
225                 230                 235                 240

aaa gat act gaa gat aaa tcc gta aaa tta tcg gat agt ttt tta gtt      768
Lys Asp Thr Glu Asp Lys Ser Val Lys Leu Ser Asp Ser Phe Leu Val
                245                 250                 255

cta aaa gat tat ttt aat ggt att tcg tcg gaa aag cct tct ggt att      816
Leu Lys Asp Tyr Phe Asn Gly Ile Ser Ser Glu Lys Pro Ser Gly Ile
            260                 265                 270

tat ggg cga tat aat tgg cat cag cta tac aat aca agt tac tat ttt      864
Tyr Gly Arg Tyr Asn Trp His Gln Leu Tyr Asn Thr Ser Tyr Tyr Phe
        275                 280                 285

ctt cga aaa gac tac tta aca gtt gaa cct cag tta cat gac tta aga      912
Leu Arg Lys Asp Tyr Leu Thr Val Glu Pro Gln Leu His Asp Leu Arg
    290                 295                 300

gaa tac tta ggt ggt tct tta aaa caa atg tca tgg gat ggt ttt tct      960
Glu Tyr Leu Gly Gly Ser Leu Lys Gln Met Ser Trp Asp Gly Phe Ser
305                 310                 315                 320

caa tta tca aaa ggt gat aaa gaa cta ttt tta aat att gtt ggg ttt     1008
Gln Leu Ser Lys Gly Asp Lys Glu Leu Phe Leu Asn Ile Val Gly Phe
                325                 330                 335

gac caa gaa aaa tta cag caa gaa tat caa caa tct gaa ttg cct aat     1056
Asp Gln Glu Lys Leu Gln Gln Glu Tyr Gln Gln Ser Glu Leu Pro Asn
            340                 345                 350

ttt gtt ttc aca ggg acg aca aca tgg gct ggt ggt gaa act aaa gaa     1104
Phe Val Phe Thr Gly Thr Thr Thr Trp Ala Gly Gly Glu Thr Lys Glu
        355                 360                 365

tat tat gct caa cag cag gta aat gtt gtt aat aat gca ata aat gag     1152
Tyr Tyr Ala Gln Gln Gln Val Asn Val Val Asn Asn Ala Ile Asn Glu
    370                 375                 380

aca agt cct tac tat cta ggt aga gag cat gat ctt ttc ttt aaa ggt     1200
Thr Ser Pro Tyr Tyr Leu Gly Arg Glu His Asp Leu Phe Phe Lys Gly
385                 390                 395                 400

cat cca aga gga gga att att aat gat att att tta ggc agt ttt aat     1248
His Pro Arg Gly Gly Ile Ile Asn Asp Ile Ile Leu Gly Ser Phe Asn
                405                 410                 415

aat atg att gat att cca gct aag gta tca ttt gaa gta ttg atg atg      1296
Asn Met Ile Asp Ile Pro Ala Lys Val Ser Phe Glu Val Leu Met Met
            420                 425                 430

aca ggg atg cta cct gat act gtt gga ggt att gca agc tct ttg tat      1344
Thr Gly Met Leu Pro Asp Thr Val Gly Gly Ile Ala Ser Ser Leu Tyr
        435                 440                 445

ttt tca ata cca gct gaa aaa gta agt ttt att gta ttt aca tcg tct      1392
Phe Ser Ile Pro Ala Glu Lys Val Ser Phe Ile Val Phe Thr Ser Ser
    450                 455                 460

gac act att aca gat aga gag gac gca tta aaa tcg cct tta gtt caa      1440
Asp Thr Ile Thr Asp Arg Glu Asp Ala Leu Lys Ser Pro Leu Val Gln
465                 470                 475                 480

gta atg atg aca ttg ggt att gta aaa gaa aaa gat gtt cta ttt tgg      1488
Val Met Met Thr Leu Gly Ile Val Lys Glu Lys Asp Val Leu Phe Trp
                485                 490                 495

tct gac tta cca gat tgt tct agt ggt gtg tgt att gct caa tat tag      1536
Ser Asp Leu Pro Asp Cys Ser Ser Gly Val Cys Ile Ala Gln Tyr
            500                 505                 510


<210>  2
<211>  511
<212>  PRT
<213>  Photobacterium leiognathi

<400>  2

Met Lys Arg Ile Phe Cys Leu Val Ser Ala Ile Leu Leu Ser Ala Cys
1               5                   10                  15


Asn Asp Asn Gln Asn Thr Val Asp Val Val Val Ser Thr Val Asn Asp
            20                  25                  30


Asn Val Ile Glu Asn Asn Thr Tyr Gln Val Lys Pro Ile Asp Thr Pro
        35                  40                  45


Thr Thr Phe Asp Ser Tyr Ser Trp Ile Gln Thr Cys Gly Thr Pro Ile
    50                  55                  60


Leu Lys Asp Asp Glu Lys Tyr Ser Leu Ser Phe Asp Phe Val Ala Pro
65                  70                  75                  80


Glu Leu Asp Gln Asp Glu Lys Phe Cys Phe Glu Phe Thr Gly Asp Val
                85                  90                  95


Asp Gly Lys Arg Tyr Val Thr Gln Thr Asn Leu Thr Val Val Ala Pro
            100                 105                 110


Thr Leu Glu Val Tyr Val Asp His Ala Ser Leu Pro Ser Leu Gln Gln
        115                 120                 125


Leu Met Lys Ile Ile Gln Gln Lys Asn Glu Tyr Ser Gln Asn Glu Arg

130                 135                 140

Phe Ile Ser Trp Gly Arg Ile Gly Leu Thr Glu Asp Asn Ala Glu Lys
145                 150                 155                 160

Leu Asn Ala His Ile Tyr Pro Leu Ala Gly Asn Asn Thr Ser Gln Glu
                165                 170                 175

Leu Val Asp Ala Val Ile Asp Tyr Ala Asp Ser Lys Asn Arg Leu Asn
            180                 185                 190

Leu Glu Leu Asn Thr Asn Thr Ala His Ser Phe Pro Asn Leu Ala Pro
        195                 200                 205

Ile Leu Arg Ile Ile Ser Ser Lys Ser Asn Ile Leu Ile Ser Asn Ile
    210                 215                 220

Asn Leu Tyr Asp Asp Gly Ser Ala Glu Tyr Val Asn Leu Tyr Asn Trp
225                 230                 235                 240

Lys Asp Thr Glu Asp Lys Ser Val Lys Leu Ser Asp Ser Phe Leu Val
                245                 250                 255

Leu Lys Asp Tyr Phe Asn Gly Ile Ser Ser Glu Lys Pro Ser Gly Ile
            260                 265                 270

Tyr Gly Arg Tyr Asn Trp His Gln Leu Tyr Asn Thr Ser Tyr Tyr Phe
        275                 280                 285

Leu Arg Lys Asp Tyr Leu Thr Val Glu Pro Gln Leu His Asp Leu Arg
    290                 295                 300

Glu Tyr Leu Gly Gly Ser Leu Lys Gln Met Ser Trp Asp Gly Phe Ser
305                 310                 315                 320

Gln Leu Ser Lys Gly Asp Lys Glu Leu Phe Leu Asn Ile Val Gly Phe
                325                 330                 335

Asp Gln Glu Lys Leu Gln Gln Glu Tyr Gln Gln Ser Glu Leu Pro Asn
            340                 345                 350

Phe Val Phe Thr Gly Thr Thr Thr Trp Ala Gly Gly Glu Thr Lys Glu
        355                 360                 365

Tyr Tyr Ala Gln Gln Gln Val Asn Val Val Asn Asn Ala Ile Asn Glu
    370                 375                 380

Thr Ser Pro Tyr Tyr Leu Gly Arg Glu His Asp Leu Phe Phe Lys Gly
385                 390                 395                 400

His Pro Arg Gly Gly Ile Ile Asn Asp Ile Ile Leu Gly Ser Phe Asn
                405                 410                 415

Asn Met Ile Asp Ile Pro Ala Lys Val Ser Phe Glu Val Leu Met Met
            420                 425                 430

Thr Gly Met Leu Pro Asp Thr Val Gly Gly Ile Ala Ser Ser Leu Tyr
        435                 440                 445

Phe Ser Ile Pro Ala Glu Lys Val Ser Phe Ile Val Phe Thr Ser Ser
    450                 455                 460

Asp Thr Ile Thr Asp Arg Glu Asp Ala Leu Lys Ser Pro Leu Val Gln
465                 470                 475                 480

Val Met Met Thr Leu Gly Ile Val Lys Glu Lys Asp Val Leu Phe Trp
                485                 490                 495

Ser Asp Leu Pro Asp Cys Ser Ser Gly Val Cys Ile Ala Gln Tyr
            500                 505                 510


<210>  3
<211>  1494
<212>  DNA
<213>  Artificial Sequence

<220>
<223>  beta-galacoside-alpha-2,6-sialyltransferase from phorobacterium l
       eiognathi (without signal peptide)

<220>
<221>  CDS
<222>  (1)..(1494)
<223>

<400>  3
atg tgt aat gat aat cag aat aca gta gat gta gtt gta tct act gtg       48
Met Cys Asn Asp Asn Gln Asn Thr Val Asp Val Val Val Ser Thr Val
1               5                   10                  15

aat gat aac gtt att gaa aat aat act tac caa gtt aaa ccc att gat       96
Asn Asp Asn Val Ile Glu Asn Asn Thr Tyr Gln Val Lys Pro Ile Asp
            20                  25                  30

act cca act act ttt gat tcc tat tct tgg ata caa aca tgc ggt act      144
Thr Pro Thr Thr Phe Asp Ser Tyr Ser Trp Ile Gln Thr Cys Gly Thr
        35                  40                  45

cca ata tta aaa gac gat gag aag tac tct ttg agt ttt gac ttt gtt      192
Pro Ile Leu Lys Asp Asp Glu Lys Tyr Ser Leu Ser Phe Asp Phe Val
    50                  55                  60

gca cct gag tta gat caa gat gaa aaa ttc tgc ttt gag ttt act ggt      240
Ala Pro Glu Leu Asp Gln Asp Glu Lys Phe Cys Phe Glu Phe Thr Gly
65                  70                  75                  80

gat gtt gat ggt aag cgt tat gtt acc caa act aat ttg act gtt gtt 288
Asp Val Asp Gly Lys Arg Tyr Val Thr Gln Thr Asn Leu Thr Val Val
                85                  90                  95

gcc cca aca cta gaa gta tat gtg gat cat gca tca ttg cca tca tta 336
Ala Pro Thr Leu Glu Val Tyr Val Asp His Ala Ser Leu Pro Ser Leu
            100                 105                 110

cag cag tta atg aaa ata atc caa cag aaa aat gag tat tca cag aat 384
Gln Gln Leu Met Lys Ile Ile Gln Gln Lys Asn Glu Tyr Ser Gln Asn
        115                 120                 125

gag cgc ttt att tct tgg gga cga att gga ctt aca gaa gat aac gca 432
Glu Arg Phe Ile Ser Trp Gly Arg Ile Gly Leu Thr Glu Asp Asn Ala
    130                 135                 140

gaa aaa tta aat gcc cat ata tat cca tta gct gga aat aac aca tca 480
Glu Lys Leu Asn Ala His Ile Tyr Pro Leu Ala Gly Asn Asn Thr Ser
145                 150                 155                 160

caa gaa ctt gta gat gca gtt att gac tat gct gac tct aaa aat cga 528
Gln Glu Leu Val Asp Ala Val Ile Asp Tyr Ala Asp Ser Lys Asn Arg
                165                 170                 175

tta aat cta gag ctt aat acg aat aca gcg cat tct ttt cca aat cta 576
Leu Asn Leu Glu Leu Asn Thr Asn Thr Ala His Ser Phe Pro Asn Leu
            180                 185                 190

gca cca ata tta cgt ata ata tca tca aag agt aat ata cta att tca 624
Ala Pro Ile Leu Arg Ile Ile Ser Ser Lys Ser Asn Ile Leu Ile Ser
        195                 200                 205

aat att aat tta tat gat gat ggt tct gca gag tat gtt aac ctt tat 672
Asn Ile Asn Leu Tyr Asp Asp Gly Ser Ala Glu Tyr Val Asn Leu Tyr
    210                 215                 220

aac tgg aaa gat act gaa gat aaa tcc gta aaa tta tcg gat agt ttt 720
Asn Trp Lys Asp Thr Glu Asp Lys Ser Val Lys Leu Ser Asp Ser Phe
225                 230                 235                 240

tta gtt cta aaa gat tat ttt aat ggt att tcg tcg gaa aag cct tct 768
Leu Val Leu Lys Asp Tyr Phe Asn Gly Ile Ser Ser Glu Lys Pro Ser
                245                 250                 255

ggt att tat ggg cga tat aat tgg cat cag cta tac aat aca agt tac 816
Gly Ile Tyr Gly Arg Tyr Asn Trp His Gln Leu Tyr Asn Thr Ser Tyr
            260                 265                 270

tat ttt ctt cga aaa gac tac tta aca gtt gaa cct cag tta cat gac 864
Tyr Phe Leu Arg Lys Asp Tyr Leu Thr Val Glu Pro Gln Leu His Asp
        275                 280                 285

tta aga gaa tac tta ggt ggt tct tta aaa caa atg tca tgg gat ggt 912
Leu Arg Glu Tyr Leu Gly Gly Ser Leu Lys Gln Met Ser Trp Asp Gly
    290                 295                 300

ttt tct caa tta tca aaa ggt gat aaa gaa cta ttt tta aat att gtt 960
Phe Ser Gln Leu Ser Lys Gly Asp Lys Glu Leu Phe Leu Asn Ile Val
305                 310                 315                 320

ggg ttt gac caa gaa aaa tta cag caa gaa tat caa caa tct gaa ttg 1008
Gly Phe Asp Gln Glu Lys Leu Gln Gln Glu Tyr Gln Gln Ser Glu Leu
                325                 330                 335

cct aat ttt gtt ttc aca ggg acg aca aca tgg gct ggt ggt gaa act 1056

Pro Asn Phe Val Phe Thr Gly Thr Thr Thr Trp Ala Gly Gly Glu Thr
            340                 345                 350

aaa gaa tat tat gct caa cag cag gta aat gtt gtt aat aat gca ata      1104
Lys Glu Tyr Tyr Ala Gln Gln Gln Val Asn Val Val Asn Asn Ala Ile
        355                 360                 365

aat gag aca agt cct tac tat cta ggt aga gag cat gat ctt ttc ttt      1152
Asn Glu Thr Ser Pro Tyr Tyr Leu Gly Arg Glu His Asp Leu Phe Phe
    370                 375                 380

aaa ggt cat cca aga gga gga att att aat gat att att tta ggc agt      1200
Lys Gly His Pro Arg Gly Gly Ile Ile Asn Asp Ile Ile Leu Gly Ser
385                 390                 395                 400

ttt aat aat atg att gat att cca gct aag gta tca ttt gaa gta ttg      1248
Phe Asn Asn Met Ile Asp Ile Pro Ala Lys Val Ser Phe Glu Val Leu
            405                 410                 415

atg atg aca ggg atg cta cct gat act gtt gga ggt att gca agc tct      1296
Met Met Thr Gly Met Leu Pro Asp Thr Val Gly Gly Ile Ala Ser Ser
            420                 425                 430

ttg tat ttt tca ata cca gct gaa aaa gta agt ttt att gta ttt aca      1344
Leu Tyr Phe Ser Ile Pro Ala Glu Lys Val Ser Phe Ile Val Phe Thr
        435                 440                 445

tcg tct gac act att aca gat aga gag gac gca tta aaa tcg cct tta      1392
Ser Ser Asp Thr Ile Thr Asp Arg Glu Asp Ala Leu Lys Ser Pro Leu
    450                 455                 460

gtt caa gta atg atg aca ttg ggt att gta aaa gaa aaa gat gtt cta      1440
Val Gln Val Met Met Thr Leu Gly Ile Val Lys Glu Lys Asp Val Leu
465                 470                 475                 480

ttt tgg tct gac tta cca gat tgt tct agt ggt gtg tgt att gct caa      1488
Phe Trp Ser Asp Leu Pro Asp Cys Ser Ser Gly Val Cys Ile Ala Gln
            485                 490                 495

tat tag                                                              1494
Tyr

<210>  4
<211>  497
<212>  PRT
<213>  Artificial Sequence

<220>
<223>  beta-galacoside-alpha-2,6-sialyltransferase from phorobacterium l
       eiognathi (without signal peptide)

<400>  4

Met Cys Asn Asp Asn Gln Asn Thr Val Asp Val Val Val Ser Thr Val
1               5                   10                  15

Asn Asp Asn Val Ile Glu Asn Asn Thr Tyr Gln Val Lys Pro Ile Asp
            20                  25                  30

Thr Pro Thr Thr Phe Asp Ser Tyr Ser Trp Ile Gln Thr Cys Gly Thr
        35                  40                  45

Pro Ile Leu Lys Asp Asp Glu Lys Tyr Ser Leu Ser Phe Asp Phe Val
    50                  55                  60

Ala Pro Glu Leu Asp Gln Asp Glu Lys Phe Cys Phe Glu Phe Thr Gly
65                  70                  75                  80

Asp Val Asp Gly Lys Arg Tyr Val Thr Gln Thr Asn Leu Thr Val Val
                85                  90                  95

Ala Pro Thr Leu Glu Val Tyr Val Asp His Ala Ser Leu Pro Ser Leu
            100                 105                 110

Gln Gln Leu Met Lys Ile Ile Gln Gln Lys Asn Glu Tyr Ser Gln Asn
        115                 120                 125

Glu Arg Phe Ile Ser Trp Gly Arg Ile Gly Leu Thr Glu Asp Asn Ala
    130                 135                 140

Glu Lys Leu Asn Ala His Ile Tyr Pro Leu Ala Gly Asn Asn Thr Ser
145                 150                 155                 160

Gln Glu Leu Val Asp Ala Val Ile Asp Tyr Ala Asp Ser Lys Asn Arg
                165                 170                 175

Leu Asn Leu Glu Leu Asn Thr Asn Thr Ala His Ser Phe Pro Asn Leu
            180                 185                 190

Ala Pro Ile Leu Arg Ile Ile Ser Ser Lys Ser Asn Ile Leu Ile Ser
        195                 200                 205

Asn Ile Asn Leu Tyr Asp Asp Gly Ser Ala Glu Tyr Val Asn Leu Tyr
    210                 215                 220

Asn Trp Lys Asp Thr Glu Asp Lys Ser Val Lys Leu Ser Asp Ser Phe
225                 230                 235                 240

Leu Val Leu Lys Asp Tyr Phe Asn Gly Ile Ser Ser Glu Lys Pro Ser
                245                 250                 255

Gly Ile Tyr Gly Arg Tyr Asn Trp His Gln Leu Tyr Asn Thr Ser Tyr
            260                 265                 270

Tyr Phe Leu Arg Lys Asp Tyr Leu Thr Val Glu Pro Gln Leu His Asp
        275                 280                 285

Leu Arg Glu Tyr Leu Gly Gly Ser Leu Lys Gln Met Ser Trp Asp Gly
    290                 295                 300

Phe Ser Gln Leu Ser Lys Gly Asp Lys Glu Leu Phe Leu Asn Ile Val
305                 310                 315                 320

Gly Phe Asp Gln Glu Lys Leu Gln Gln Glu Tyr Gln Gln Ser Glu Leu
                325                 330                 335

Pro Asn Phe Val Phe Thr Gly Thr Thr Thr Trp Ala Gly Gly Glu Thr
            340                 345                 350

Lys Glu Tyr Tyr Ala Gln Gln Gln Val Asn Val Val Asn Asn Ala Ile
        355                 360                 365

Asn Glu Thr Ser Pro Tyr Tyr Leu Gly Arg Glu His Asp Leu Phe Phe
    370                 375                 380

Lys Gly His Pro Arg Gly Gly Ile Ile Asn Asp Ile Ile Leu Gly Ser
385                 390                 395                 400

Phe Asn Asn Met Ile Asp Ile Pro Ala Lys Val Ser Phe Glu Val Leu
                405                 410                 415

Met Met Thr Gly Met Leu Pro Asp Thr Val Gly Gly Ile Ala Ser Ser
            420                 425                 430

Leu Tyr Phe Ser Ile Pro Ala Glu Lys Val Ser Phe Ile Val Phe Thr
        435                 440                 445

Ser Ser Asp Thr Ile Thr Asp Arg Glu Asp Ala Leu Lys Ser Pro Leu
    450                 455                 460

Val Gln Val Met Met Thr Leu Gly Ile Val Lys Glu Lys Asp Val Leu
465                 470                 475                 480

Phe Trp Ser Asp Leu Pro Asp Cys Ser Ser Gly Val Cys Ile Ala Gln
                485                 490                 495

Tyr

<210>  5
<211>  11
<212>  DNA
<213>  Artificial Sequence

<220>
<223>  Primer: 2,6 consensus 691-701F

<400>  5
gatgatggtt c                                                            11

<210>  6
<211>  11
<212>  DNA
<213>  Artificial Sequence

<220>
<223>  Primer: 2,6 consensus 1300-1310R

<400>  6
gtcatcatca a                                                            11


<210>  7
<211>  15
<212>  DNA
<213>  Artificial Sequence

<220>
<223>  Primer: 2,6 consensus 688-702F

<400>  7
taygatgatg gttcw                                                        15


<210>  8
<211>  24
<212>  DNA
<213>  Artificial Sequence

<220>
<223>  Primer: 2,6 consensus 1288-1311R

<220>
<221>  misc_feature
<222>  (13)..(13)
<223>  n stands for any base


<400>  8
ygtcatcatc aanacytcaa atga                                              24


<210>  9
<211>  20
<212>  DNA
<213>  Artificial Sequence

<220>
<223>  Primer: SHIZ-119-26 412-431F

<400>  9
gagtattcac agaatgagcg                                                   20


<210>  10
<211>  20
<212>  DNA
<213>  Artificial Sequence

<220>
<223>  Primer: SHIZ-119-26 521-540F

<400>  10
cacaagaact tgtagatgca                                                   20

<210>  11
<211>  20
<212>  DNA
<213>  Artificial Sequence

<220>
<223>  Primer: SHIZ-119-26 325-344F

<400>  11
gttgttgccc caacactaga                                                    20


<210>  12
<211>  20
<212>  DNA
<213>  Artificial Sequence

<220>
<223>  Primer: SHIZ-119-26 640-659F

<400>  12
ctaggtagag agcatgatct                                                    20


<210>  13
<211>  20
<212>  DNA
<213>  Artificial Sequence

<220>
<223>  Primer: SHIZ-119-26 671-690F

<400>  13
gtcatccaag aggaggaatt                                                    20


<210>  14
<211>  36
<212>  DNA
<213>  Artificial Sequence

<220>
<223>  Primer: SHIZ119 N0 Bsp

<400>  14
gcgcgtcatg aaaagaatat tttgtttagt ctctgc                                  36


<210>  15
<211>  30
<212>  DNA
<213>  Artificial Sequence

<220>
<223>  Primer: SHIZ119 C0 Bam

<400>  15
attaaggatc cctaatattg agcaatacac                                         30


<210>  16
<211>  30
<212>  DNA
<213>  Artificial Sequence

<220>
<223>  Primer: SHIZ119 N1 Pci

<400>  16
gggacatgtg taatgataat cagaatacag                                        30


<210>  17
<211>  8
<212>  PRT
<213>  Artificial Sequence

<220>
<223>  FLAG tag

<400>  17
Asp Tyr Lys Asp Asp Asp Asp Lys                                         8